# EUROPÄISCHE PATENTSCHRIFT

(11) **EP 0 381 728 B1**
(45) Veröffentlichungstag und Bekanntmachung des Hinweises auf die Patenterteilung: **08.12.1993**
(21) Anmeldenummer: 89908422.2
(22) Anmeldetag: 26.07.1989
(51) Int. Cl.: C09K 19/00

(54) **DIFLUORMETHYLVERBINDUNGEN**
DIFLUOROMETHYL COMPOUNDS
COMPOSES DE DIFLUOROMETHYLE

(30) Priorität: 27.07.1988 DE 3825425; 24.03.1989 DE 3909802
(43) Veröffentlichungstag der Anmeldung: 16.08.1990
(73) Patentinhaber: MERCK PATENT GmbH, D-64271 Darmstadt (DE)
(72) Erfinder: DORSCH, Dieter, D-6100 Darmstadt (DE); BARTMANN, Ekkehard, D-6106 Erzhausen (DE); KURMEIER, Hans-Adolf, D-6104 Seeheim (DE); FINKENZELLER, Ulrich, D-6831 Plankstadt (DE); WEBER, Georg, D-6106 Erzhausen (DE); PLACH, Herbert, D-6100 Darmstadt (DE); POETSCH, Eike, D-6109 Mühltal (DE)
(86) Internationale Anmeldenummer: EP8900821
(87) Internationale Veröffentlichungsnummer: WO9001056

(56) Entgegenhaltungen:
- EP-A- 255 236
- MOL. CRYST. LIQ. CRYST., Band 47, Nr. 1/2, 1978, Gordon & Breach Science Publishers Ltd.; V.V. TITOV et al.: "Synthesis and mesomorphism of aryl p-fluoralkyl (alkoxy) benzoates", pp. 1-5

## Beschreibung

Die Erfindung betrifft Difluormethylverbindungen der Formel I,

R-(A¹-Z¹)ₘ-A²-Q-CHF₂ I

worin
- R: Halogen, -CN, -NCS, einen geradkettigen Alkyl- oder Alkenylrest mit 1 bis 15 C-Atomen, wobei in diesen Resten auch eine oder mehrere CH₂-Gruppen jeweils unabhängig voneinander durch -O-, -S-, 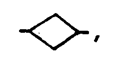 -CO-, CO-O, -O-CO- oder -O-CO-O- so ersetzt sein können, daß O-Atome nicht direkt miteinander verknüpft sind, oder einen geradkettigen Perfluordlkylrest, in dem und Fluor partiell durch H ersetzt sein kann,
- A¹ und A²: jeweils unabhängig voneinander einen
(a) trans-1,4-Cyclohexylenrest, worin auch eine oder mehrere nicht benachbarte CH₂-Gruppen durch -o- und/oder -S- ersetzt sein können,
(b) 1,4-Phenylenrest, worin auch eine oder zwei CH-Gruppen durch N ersetzt sein können,
(c) Rest aus der Gruppe 1,4-Cyclohexenylen, 1,4-Bicyclo(2,2,2)-octylen, Piperidin-1,4-diyl, Naphthalin-2,6-diyl, Decahydronaphthalin-2,6-diyl und 1,2,3,4-Tetrahydronaphthalin-2,6-diyl,
wobei die Reste (a) und (b) durch CN oder Fluor substituiert sein können,
- Z¹: jeweils unabhängig voneinander -CO-O-, -O-CO-, -CH₂O-, -OCH₂-, -CH₂CH₂-, -CH=CH-, -C≡C- oder eine Einfachbindung,
- m: 1, 2 oder 3 und
- Q: Alkylen mit 2 bis 6 C-Atomen, worin auch eine CH₂-Gruppe durch -O-, -S-, -CO-O- oder -O-CO- ersetzt sein kann, -O-, -S-, -CH₂-, -CO-O-, -O-CO- oder eine Einfachbindung
bedeutet, mit der Maßgabe, daß Q Alkylen mit 2 bis 6 C-Atomen, worin auch eine CH₂-Gruppe durch -O-, -S-, -CO-O- oder -O-CO- ersetzt sein kann, -S-, -CH₂-, -CO-O-, -O-CO- oder eine Einfachbindung bedeutet, falls 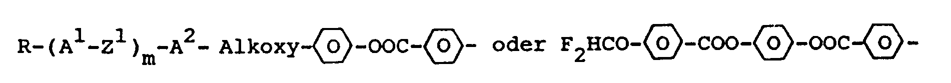
bedeutet.

Die Erfindung betrifft weiterhin die Verwendung dieser Verbindungen als Komponenten flüssigkristalliner Medien sowie Flüssigkristall- und elektrooptische Anzeigeelemente, die die erfindungsgemäßen flüssigkristallinen Medien enthalten.

Die Verbindungen der Formel I können als Komponenten flüssigkristalliner Medien verwendet werden, insbesondere für Displays, die auf dem Prinzip der verdrillten Zelle, dem Guest-Host-Effekt, dem Effekt der Deformation aufgerichteter Phasen oder dem Effekt der dynamischen Streuung beruhen.

Der Erfindung lag die Aufgabe zugrunde, neue stabile flüssigkristalline oder mesogene Verbindungen aufzufinden, die als Komponenten flüssigkristalliner Medien geeignet sind und insbesondere gleichzeitig eine vergleichsweise geringe Viskosität besitzen sowie eine relativ hohe dielektrische Anisotropie.

Es wurde nun gefunden, daß Verbindungen der Formel I als Komponenten flüssigkristalliner Medien vorzüglich geeignet sind. Insbesondere verfügen sie über vergleichsweise niedere Viskositäten. Mit ihrer Hilfe lassen sich stabile flüssigkristalline Medien mit breitem Mesophasenbereich und vorteilhaften Werten für die optische und dielektrische Anisotropie erhalten.

Verbindungen mit flüssigkristallinen Eigenschaften, in denen terminal eine OCHF₂-Gruppe gebunden ist, sind bereits bekannt. Einerseits wurden an Schiff'schen Basen Kristallstrukturuntersuchungen vorgenommen [S.V. Sereda et al. in Kristallografiya, 32 (5), 1165 (1987) und ibid. 33 (1) 118 (1988)]. Andererseits sind von V.V. Titov et al. in Mol. Cryst. Liq. Cryst. 47 (1-2), 1 (1978) Benzoesäureester beschrieben, die in p-Position eine OCHF₂-Gruppe tragen. Die bekannten Verbindungen sind jedoch instabil oder in kommerziellen Displays nicht einsetzbar. Ferner sind aus der EP-A- 0 255 236 Verbindungen mit fluorhaltigen Seitenketten bekannt. Die dort beschriebenen Verbindungen besitzen jedoch stets eine chirale Flügelgruppe und weisen daher aufgrund ihrer Struktur getiltet smektische Mesophasen auf und sind für die Verwendung in ferroelektrischen Flüssigkristallmischungen vorgesehen. Sie haben also völlig unterschiedliche Eigenschaften und besitzen einen anderen Verwendungszweck als die erfindungsgemäßen Verbindungen.

Mit der Bereitstellung von Verbindungen der Formel I wird außerdem ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Medien zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie stabil.

Gegenstand der Erfindung sind somit die Verbindungen der Formel I sowie die Verwendung dieser Verbindungen als Komponenten flüssigkristalliner Medien. Gegenstand der Erfindung sind ferner flüssigkristalline Medien mit einem Gehalt an mindestens einer Verbindung der Formel I sowie Flüssigkristallanzeigeelemente, insbesondere elektroopische Anzeigeelemente, die derartige Medien enthalten.

Der Einfachheit halber bedeuten im folgenden X Q-CHF₂, Cyc einen 1,4-Cyclohexylenrest, Che einen 1,4-Cyclohexenylenrest, Dio einen 1,3-Dioxan-2,5-diylrest, Dit einen 1,3-Dithian-2,5-diylrest, Phe einen 1,4-Phenylenrest, Pyd einen Pyridin-2,5-diylrest, Pyr einen Pyrimidin-2,5-diylrest, Pip einen Piperidin-1,4-diylrest, Nap einen Naphthalin-2,6-diylrest, Dec und Tet einen Deca- bzw. 1,2,3,4-Tetrahydronaphthalinrest und Bi einen Bicyclo(2,2,2)-octylenrest, wobei Cyc und/oder Phe unsubstituiert oder ein- oder zweifach durch F oder CN substituiert sein können.

Die Verbindungen der Formel I umfassen dementsprechend Verbindungen mit zwei Ringen der Teilformeln Ia und Ib:

R-A¹-A²-X Ia

R-A¹-Z¹-A²-X Ib

Verbindungen mit drei Ringen der Teilformeln Ic bis If:

R-A¹-A¹-A²-X Ic

R-A¹-Z¹-A¹-Z¹-A²-X Id

R-A¹-Z¹-A¹-A²-X Ie

R-A¹-A¹-Z¹-A²-X If

sowie Verbindungen mit vier Ringen der Teilformeln Ig bis In:

R-A¹-A¹-A¹-A²-X Ig

R-A¹-Z¹-A¹-A¹-A²-X Ih

R-A¹-A¹-Z¹-A¹-A²-X Ii

R-A¹-A¹-A¹-Z¹-A²-X Ij

R-A¹-Z¹-A¹-Z¹-A¹-A²-X Ik

R-A¹-Z¹-A¹-A¹-A¹-Z¹-A²-X Il

R-A¹-A¹-Z¹-A¹-Z¹-A²-X Im

R-A¹-Z¹-A¹-Z¹-A¹-Z¹-A²-X In

Darunter sind besonders diejenigen der Teilformeln Ia, Ib, Ic, Id, Ie, If, Ii und Il bevorzugt.

Die bevorzugten Verbindungen der Teilformel Ia umfassen diejenigen der Teilformeln Iaa bis Iah:

R-Phe-Phe-X Iaa

R-Phe-Cyc-X Iab

R-Dio-Phe-X Iac

R-Pyr-Phe-X Iad

R-Pyd-Phe-X Iae

R-Cyc-Phe-X Iaf

R-Cyc-Cyc-X Iag

R-Che-Phe-X Iah

Darunter sind diejenigen der Formeln Iaa, Iab, Iac, Iad, Iaf und Iag besonders bevorzugt.

Die bevorzugten Verbindungen der Teilformel Ib umfassen diejenigen der Teilformeln Iba bis Ibm:

R-Phe-CH₂CH₂-Phe-X Iba

R-Phe-OCH₂-Phe-X Ibb

R-Cyc-CH₂CH₂-Phe-X Ibc

R-Cyc-CH₂-CH₂-Cyc-X Ibd

R-Cyc-COO-Phe-X Ibe

R-Cyc-COO-Cyc-X Ibf

R-A¹-CH₂CH₂-Phe-X Ibg

R-A¹-CH₂CH₂-Cyc-X Ibh

R-A¹-CH₂O-Phe-X Ibi

R-A¹-OCH₂-Phe-X Ibj

R-A¹-COO-Phe-X Ibk

R-A¹-OOC-Phe-X Ibl

R-Che-CH₂CH₂-Phe-X Ibm

Die bevorzugten Verbindungen der Teilformel Ic umfassen diejenigen der Teilformeln Ica bis Icq:

R-Phe-Phe-Phe-X Ica

R-Phe-Phe-Cyc-X Icb

R-Phe-Dio-Phe-X Icc

R-Cyc-Cyc-Phe-X Icd

R-Phe-Cyc-Phe-X Ice

R-Cyc-Cyc-Cyc-X Icf

R-Pyd-Phe-Phe-X Icg

R-Pyr-Phe-Phe-X Ich

R-Phe-Pyr-Phe-X Ici

R-Cyc-Pyr-Phe-X Icj

R-Cyc-Phe-Phe-X Ick

R-Cyc-Phe-Cyc-X Icl

R-Dio-Phe-Phe-X Icm

R-Che-Phe-Phe-X Icn

R-Phe-Che-Phe-X Ico

R-Che-Cyc-Phe-X Icp

R-Cyc-Che-Phe-X Icq

Darunter sind diejenigen der Formeln Ica, Icc, Icd, Ice, Ici und Icj besonders bevorzugt.

Die bevorzugten Verbindungen der Teilformel Id umfassen diejenigen der Teilformeln Ida bis Idn:

R-Phe-Z¹-Phe-Z¹-Phe-X Ida

R-Phe-Z¹-Phe-Z¹-Cyc-X Idb

R-Phe-Z¹-Dio-Z¹-Phe-X Idc

R-Cyc-Z¹-Cyc-Z¹-Phe-X Idd

R-Cyc-Z¹-Cyc-Z¹-Cyc-X Ide

R-Pyd-Z¹-Phe-Z¹-Phe-X Idf

R-Phe-Z¹-Pyd-Z¹-Phe-X Idg

R-Pyr-Z¹-Phe-Z¹-Phe-X Idh

R-Phe-Z¹-Pyr-Z¹-Phe-X Idi

R-Phe-Z¹-Cyc-Z¹-Phe-X Idj

R-Cyc-Z¹-Phe-Z¹-Cyc-X Idk

R-Cyc-Z¹-Phe-Z¹-Phe-X Idl

R-Dio-Z¹-Phe-Z¹-Phe-X Idm

R-Che-Z¹-Phe-Z¹-Phe-X Idn

Die bevorzugten Verbindungen der Teilformel Ie umfassen diejenigen der Teilformeln Iea bis Iem:

R-Pyr-Z¹-Phe-Phe-X Iea

R-Dio-Z¹-Phe-Phe-X Ieb

R-Phe-Z¹-Phe-Phe-X Iec

R-Cyc-Z¹-Phe-Phe-X Ied

R-Cyc-Z¹-Phe-Cyc-X Iee

R-Phe-Z¹-Cyc-Phe-X Ief

R-Cyc-Z¹-Cyc-Phe-X Ieg

R-Cyc-Z¹-Cyc-Cyc-X Ieh

R-Phe-Z¹-Dio-Phe-X Iei

R-Pyd-Z¹-Phe-Phe-X Iej

R-Phe-Z¹-Pyr-Phe-X Iek

R-Cyc-Z¹-Pyr-Phe-X Iel

R-Phe-Z¹-Che-Phe-X Iem

Die bevorzugten Verbindungen der Teilformel If umfassen diejenigen der Teilformeln Ifa bis Ifr:

R-Pyr-Phe-Z¹-Phe-X Ifa

R-Pyr-Phe-OCH₂-Phe-X Ifb

R-Phe-Phe-Z¹-Phe-X Ifc

R-Phe-Phe-OOC-Phe-X Ifd

R-Phe-Phe-Z¹-Cyc-X Ife

R-Cyc-Cyc-Z¹-Phe-X Iff

R-Cyc-Cyc-Z¹-Cyc-X Ifg

R-Cyc-Cyc-CH₂CH₂-Phe-X Ifh

R-Pyd-Phe-Z¹-Phe-X Ifi

R-Dio-Phe-Z¹-Phe-X Ifj

R-Phe-Cyc-Z¹-Phe-X Ifk

R-Phe-Cyc-Z¹-Cyc-X Ifl

R-Phe-Pyd-Z¹-Phe-X Ifm

R-Che-Phe-Z¹-Phe-X Ifn

R-Phe-Che-Z¹-Phe-X Ifo

R-Cyc-Phe-Z¹-Phe-X Ifp

R-Cyc-Phe-OOC-Phe-X Ifq

R-Cyc-Phe-Z¹-Cyc-X Ifr

Die bevorzugten Verbindungen der Teilformeln Ig bis In umfassen diejenigen der Teilformeln Io bis Iy:

R-Cyc-Phe-Cyc-Phe-X Io

R-Cyc-Cyc-Phe-Phe-X Ip

R -Cyc-Phe-Phe-Cyc-X Iq

R-A¹-CH₂O-A¹-A¹-Phe-X Ir

R-Cyc-Cyc-Z¹-A¹-Phe-X Is

R-Cyc-Cyc-Z¹-Y¹-Cyc-X It

R-A¹-A¹-A¹-CH₂CH₂-Phe-X Iu

R-Phe-Z¹-Phe-Z¹-Dio-Phe-X Iv

R-Phe-Z¹-Phe-Phe-Z¹-Phe-X Iw

R-A¹-COO-A¹-COO-A¹-Phe-X Ix

R-A¹-A¹-COO-A¹-Z¹-Phe-X Iy

In den Verbindungen der vor- und nachstehenden Formeln bedeutet X vorzugsweise Q-CHF₂, worin Q Alkylen mit 2 bis 4 C-Atomen bedeutet, worin auch eine CH₂-Gruppe durch -O-, -CO-O- oder -O-CO- ersetzt sein kann. Weiterhin bedeutet Q vorzugsweise eine Einfachbindung, -O-, -S-, -CH₂- und -CO-O-, insbesondere bevorzugt sind -O- und -S-.

Wenn Q eine Einfachbindung bedeutet, so sind diejenigen Verbindungen der Formel I bevorzugt, in denen X an einen cycloaliphathischen Ring gebunden ist. Bedeutet Q -O- oder -S-, so sind diejenigen Verbindungen der Formel I bevorzugt, in denen X an einen aromatischen Ring gebunden ist.

R bedeutet vorzugsweise Alkyl, ferner Alkoxy. Ferner bedeutet R vorzugsweise einen Perfluoralkylrest, in dem auch Fluor partiell durch Wasserstoff ersetzt sein kann.

Insbesondere bevorzugt sind dabei Reste R mit der allgemeinen Formel -CH₂-CₙF₂ₙ₊₁ und CₚF₂ₚ₊₁ mit n gleich 1 bis 14 und p gleich 1 bis 15. A¹ und/oder A² bedeuten bevorzugt Phe, Cyc, Che, Pyr oder Dio. Besonders bevorzugt bedeutet A¹ und A² Phe oder Cyc. Ferner sind solche Verbindungen der Formel I bevorzugt, in denen neben Phe und/oder Cyc zusätzlich ein weiterer Ring, ausgewählt aus der Gruppe Che, Pip, Pyr, Pyd, Dio, Dit, Bi, Nap, Dec oder Tet enthalten ist.

Bevorzugt sind auch Verbindungen der Formel I sowie aller Teilformeln, in denen A¹ und/oder A² ein- oder zweifach durch F oder einfach durch CN substituiertes 1,4-Phenylen bedeutet. Insbesondere sind dies 2-Fluor-1,4-phenylen, 3-Fluor-1,4-phenylen und 2,3-Difluor-1,4-phenylen sowie 2-Cyan-1,4-phenylen und 3-Cyan-1,4-phenylen. Unter den durch F substituierten 1,4-Phenylenresten sind insbesondere 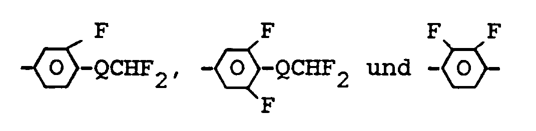
bevorzugt.

Falls ein Rest (a) durch F oder CN substitiuiert ist, so ist 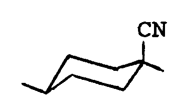
bevorzugt.

Z¹ bedeutet bevorzugt eine Einfachbindung, -CO-O-, -O-CO- und -CH₂CH₂-, in zweiter Linie bevorzugt -CH₂O- und -OCH₂-. Besonders bevorzugt sind Verbindungen, in denen nicht mehr als ein, höchstens zwei Brückenglieder Z¹ (Z¹ ungleich Einfachbindung) enthalten sind.

m bedeutet vorzugsweise 1 oder 2.

Falls R Halogen bedeutet, so bedeutet R vorzugsweise F, Cl, Br, ferner auch I.

Falls R einen Alkylrest und/oder einen Alkoxyrest bedeutet, so ist dieser geradkettig und hat Vorzugsweise 2, 3, 4, 5, 6 oder 7 C-Atome und bedeutet demnach bevorzugt Ethyl, Propyl, Butyl, Pentyl, Hexyl, Heptyl, Ethoxy, Propoxy, Butoxy, Pentoxy, Hexoxy oder Heptoxy, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy oder Tetradecoxy.

Oxaalkyl bedeutet vorzugsweise geradkettiges 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl.

Falls R einen Alkylrest bedeutet, in dem eine CH₂-Gruppe durch -CH=CH- ersetzt ist, so ist dieser geradkettig und hat vorzugsweise 2 bis 10 C-Atome. Er bedeutet demnach besonders Vinyl, Prop-1-, oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Falls R einen Alkylrest bedeutet, in dem eine CH₂-Gruppe durch -O- und eine durch -CO- ersetzt ist, so sind diese bevorzugt benachbart. Somit beeinhalten diese eine Acyloxygruppe -CO-O- oder eine Oxycarbonylgruppe -O-CO-. Vorzugsweise haben sie 2 bis 6 C-Atome.

Sie bedeuten demnach besonders Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 3-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)ethyl, 3-(Methoxycarbonyl)propyl, 3-(Ethoxycarbonyl)propyl, 4-(Methoxycarbonyl)-butyl.

Falls R einen Alkylrest bedeutet, in dem eine CH₂-Gruppe durch -CR=CH- und eine benachbarte CH₂-Gruppe durch CO oder CO-O oder O-CO- ersetzt ist, hat er vorzugsweise 4 bis 13 C-Atome. Er bedeutet demnach besonders Acryloyloxymethyl, 2-Acryloyloxyethyl, 3-Acryloyloxypropyl, 4-Acryloyloxybutyl, 5-Acryloyloxypentyl, 6-Acryloyloxyhexyl, 7-Acryloyloxyheptyl, 8-Acryloyloxyoctyl, 9-Acryloyloxynonyl, 10-Acryloyloxydecyl, Methacryloyloxymethyl, 2-Methacryloyloxyethyl, 3-Methacryloyloxypropyl, 4-Methacryloyloxybutyl, 5-Methacryloyloxypentyl, 6-Methacryloyloxyhexyl, 7-Methacryloyloxyheptyl, 8-Methacryloyloxyoctyl, 9-Methacryloyloxynonyl.

Verbindungen der Formel I, die über für Polymerisationsreaktionen geeignete Flügelgruppen R verfügen, eignen sich zur Darstellung flüssigkristalliner Polymerer.

Verbindungen der Formel I mit S_{A}-Phasen eignen sich beispielsweise für thermisch adressierte Displays.

Verbindungen der Formel I, die über für Polykondensationen geeignete Flügelgruppen R verfügen, eignen sich zur Darstellung flüssigkristalliner Polykondensate.

Unter diesen Verbindungen der Formel I sowie den Unterformeln sind diejenigen bevorzugt, in denen mindestens einer der darin enthaltenden Reste eine der angegebenen bevorzugten Bedeutungen hat.

In den Verbindungen der Formel I sind diejenigen Stereoisomeren bevorzugt, in denen die Ringe Cyc und Piperidin trans-1,4-disubstituiert sind. Diejenigen der vorstehend genannten Formeln, die eine oder mehrere Gruppen Pyd, Pyr und/oder Dio enthalten, umschließen jeweils die beiden 2,5-Stellungsisomeren.

Die 1,4-Cyclohexenylen-Gruppe hat vorzugsweise folgende Strukturen: 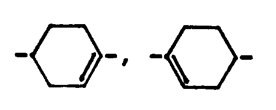
Die Verbindungen der Formel I werden nach an sich bekannten Methoden dargestellt, wie sie in der Literatur (z.B. in den Standardwerken wie Houben-Weyl, Methoden der Organischen Chemie, Georg-Thieme-Verlag, Stuttgart Bd IX, S. 867 ff.) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die erfindungsgemäßen Aryldiflourmethylether können z.B. hergestellt, indem man Hydroxyarylverbindungen mit Chlordifluormethan unter Reaktionsbedingungen umsetzt, die für die Reimer-Tiemann-Reaktion zwischen Phenolen und Chloroform geeignet sind [T.G. Miller, J.W. Thanassi, J. Org. Chem. 25, 2009 (1960)].

Die Veretherung kann in bekannter Weise in aprotischen, stark polaren Solventien (JP-OS 59157041) sowie in wäßrigen oder auch in nahezu wasserfreien Medien durchgeführt werden, indem man beispielweise die Alkoholatbildung in wäßrig-organischer Phase (z.B. Tetrahydrofuran/Wasser) durchführt, vor der eigentlich Veretherung denn jedoch das Wasser zum Überwiegenden Teil azeotrop entfernt.

Erfindungsgemäße Difluoralkylarylether können beispielsweise auch hergestellt werden, indem man z.B. entsprechende Nitro- oder Fluorobenzole, die zusätzlich mindestens einen elektronensiehenden Substituenten tragen, direkt mit Alkalidifluoralkoxyverbindungen umsetzt, wobei F bzw. NO₂ gegen den Difluoralkoxyrest substituiert wird [J. P. Idoux et al., J.Org. Chem. 50, 1976 (1985)].

Difluormethylverbindungen der Formel I können z.B. hergestellt werden, indem man Aldehyde mit Dialkylaminoschwefeltrifluorid, beispielsweise DAST (Diethylaminoschwefeltrifluorid) umsetzt [W.J. Middleton, J.Org. Chem. 40, 574 (1975)].

Difluormethylthioverbindungen lassen sich nach gleicher Methode herstellen wie Difluormethoxyverbindungen (z.B. nach L.N. Sedova et al., Zh. Org. Khim. 6, (1970) 568).

Ausgangstoffe können gewünschtenfalls auch in situ gebildet werden, derart, daß man sie aus dem Reaktionsgemisch nicht isoliert, sondern sofort weiter zu den Verbindungen der Formel I umsetzt.

So können die Verbindungen der Formel I hergestellt werden, indem man eine Verbindung, die sonst der Formel I entspricht, aber an Stelle von H-Atomen eine oder mehrere reduzierbare Gruppen und/oder C-C-Bindungen enthält, reduziert.

Als reduzierbare Gruppen kommen vorzugsweise Carbonylgruppen in Betracht, insbesondere Ketogruppen, ferner z.B. freie oder veresterte Hydroxygruppen oder aromatisch gebundene Halogenatome. Bevorzugte Ausgangsstoffe für die Reduktion entsprechen der Formel I, können aber an Stelle eines Cyclohexanringes einen Cyclohexenring oder Cyclohexanonring und/oder an Stelle einer -CH₂CH₂-Gruppe eine -CH=CH-Gruppe und/oder an Stelle einer -CH₂-Gruppe eine -CO-Gruppe und/oder an Stelle eines H-Atoms eine freie oder eine funktionell (z.B. in Form ihres p-Toluolsulfonats) abgewandelte OH-Gruppe enthalten.

Die Reduktion kann z.B. erfolgen durch katalytische Hydrierung bei Temperaturen zwischen etwa O° und etwa 200° sowie Drucken zwischen etwa 1 und 200 bar in einem inerten Lösungsmittel, z.B. einem Alkohol wie Methanol, Ethanol oder Isopropanol, einem Ether wie Tetrahydrofuran (THF) oder Dioxan, einem Ester wie Ethylacetat, einer Carbonsäure wie Essigsäure oder einem Kohlenwasserstoff wie Cyclohexan. Als Katalysatoren eignen sich zweckmäßig Edelmetalle wie Pt oder Pd, die in Form von Oxiden (z.B. PtO₂, PdO), auf einem Träger (z.B. Pd auf Kohle, Calciumcarbonat oder Strontiumcarbonat) oder in feinverteilter Form eingesetzt werden können.

Ketone können auch nach den Methoden von Clemmensen (mit Zink, amalgamiertem Zink oder Zinn und Salzsäure, zweckmäßig in wäßrig-alkoholischer Lösung oder in heterogener Phase mit Wasser/Toluol bei Temperaturen zwischen etwa 80 und 120°) oder Wolff-Kishner (mit Hydrazin, zweckmäßig in Gegenwart von Alkali wie KOH oder NaOH in einem hochsiedenden Lösungsmittel wie Diethylenglykol oder Triethylenglykol bei Temperaturen zwischen etwa 100 und 200°) zu den entsprechenden Verbindungen der Formel I, die Alkylgruppen und/oder -CH₂CH₂-Brücken enthalten, reduziert werden.

Weiterhin sind Reduktionen mit komplexen Hydriden möglich. Beispielsweise können Arylsulfonyloxygruppen mit LiAlH₄ reduktiv entfernt werden, insbesondere p-Toluolsulfonyloxymethylgruppen zu Methylgruppen reduziert werden, zweckmäßig in einem inerten Lösungsmittel wie Diethylether oder THF bei Temperaturen zwischen etwa 0 und 100°. Doppelbindungen können mit NaBH₄ oder Tributylzinnhydrid in Methanol hydriert werden.

Verbindungen der Formel I, die ansonsten der Formel I entsprechen, aber an Stelle von 1,4-Phenylenresten 1,4-Cyclohexenylenreste besitzen, können zum Beispiel mit DDQ (Dichlordicyanobenzochinon) in einem geeigneten Lösungsmittel oxidiert werden.

Ester der Formel I können auch durch Veresterung entsprechender Carbonsäuren (oder ihrer reaktionsfähigen Derivate) mit Alkoholen bzw. Phenolen (oder ihren reaktionsfähigen Derivaten) oder nach der DCC-Methode (DCC = Dicyclohexylcarbodiimid) erhalten werden.

Die entsprechenden Carbonsäuren und Alkohole bzw. Phenole sind bekannt oder können in Analogie zu bekannten Verfahren hergestellt werden.

Thiophenole können beispielsweise hergestellt werden, indem man entsprechende Benzolderivate mit Chlorsulfonsäure umsetzt und anschließend beispielsweise mit Zink/verdünnter Salzsäure reduziert, oder indem man entsprechende Phenolderivate mit Dimethylcarbamoylchlorid umsetzt und anschließend umlagert, wie in der DE 34 34 335 beschrieben.

Als reaktionsfähige Derivate der genannten Carbonsäuren eignen sich insbesondere die Säurehalogenide, vor allem die Chloride und Bromide, ferner die Anhydride, z.B. auch gemischte Anhydride, Azide oder Ester, insbesondere Alkylester mit 1-4 C-Atomen in der Alkylgruppe.

Als reaktionsfähige Derivate der genannten Alkohole bzw. Phenole kommen insbesondere die entsprechenden Metallalkoholate bzw. Phenolate, vorzugsweise eines Alkalimetalls wie Natrium oder Kalium, in Betracht.

Die Veresterung wird vorteilhaft in Gegenwart eines inerten Lösungsmittels durchgeführt. Gut geeignet sind insbesondere Ether wie z. B. Diethylether, Di-n-butylether, THF, Dioxan oder Anisol, Ketone wie z. B. Aceton, Butanon oder Cyclohexanon, Amide wie z. B. DMF oder Phosphorsäurehexamethyltriamid, Kohlenwasserstoffe wie z.B. Benzol, Toluol oder Xylol, Halogenkohlenwasserstoffe wie z.B. Tetrachlorkohlenstoff, Dichlormethan oder Tetrachlorethylen und Sulfoxide wie z. B. Dimethylsulfoxid oder Sulfolan.

In einem weiteren Verfahren zur Herstellung der Verbindungen der Formel I setzt man ein Arylhalogenid mit einem Olefin um in Gegenwart eines tertiären Amins und eines Palladiumkatalysators (vgl. R.F. Heck, Acc. Chem. Res. 12 (1979) 146). Geeignete Arylhalogenide sind beispielsweise Chloride, Bromide und Iodide, insbesondere Bromide und Iodide. Die für das Gelingen der Kupplungsreaktion erforderlichen tertiären Amine, wie z.B. Triethylamin, eignen sich auch als Lösungsmittel. Als Palladiumkatalysatoren sind beispielsweise dessen Salze, insbesondere Pd(II)-acetat, mit organischen Phosphor(III)-Verbindungen wie z.B. Triarylphosphanen geeignet. Man kann dabei in Gegenwart oder Abwesenheit eines inerten Lösungsmittels bei Temperaturen zwischen etwa 0° und 150°, vorzugsweise zwischen 20° und 100°, arbeiten; als Lösungsmittel kommen z.B. Nitrile wie Acetonitril oder Kohlenwasserstoffe wie Benzol oder Toluol in Betracht. Die als Ausgangsstoffe eingesetzten Arylhalogenide und Olefine sind vielfach im Handel erhältlich oder können nach literaturbekannten Verfahren hergestellt werden, beispielsweise durch Halogenierung entsprechender Stammverbindungen bzw. durch Eliminierungsreaktionen an entsprechenden Alkoholen oder Halogeniden.

Auf diese Weise sind beispielsweise Stilbenderivate herstellbar. Die Stilbene können weiterhin hergestellt werden durch Umsetzung eines 4-substituierten Benzaldehyds mit einem entsprechenden Phosphorylid nach Wittig. Man kann aber auch Tolane der Formel I herstellen, indem man anstelle des Olefins monosubstituiertes Acetylen einsetzt (Synthesis 627 (1980) oder Tetrahedron Lett. 27, 1171 (1986)).

Weiterhin können zur Kopplung von Aromaten Arylhalogenide mit Arylzinnverbindungen umgesetzt werden. Bevorzugt werden diese Reaktionen unter Zusatz eines Katalysators wie z.B. eines Palladium(0)komplexes in inerten Lösungsmitteln wie Kohlenwasserstoffen bei hohen Temperaturen, z.B. in siedendem Xylol, unter Schutzgas durchgeführt.

Kopplungen von Alkinyl-Verbindungen mit Arylhalogeniden können analog dem von A.O. King, E. Negishi, F.J. Villani und A. Silveira in J.Org.Chem. 43, 358 (1978) beschriebenen Verfahren durchgeführt werden.

Tolane der Formel I können auch über die Fritsch-Buttenberg-Wiechell-Umlagerung (Ann. 279, 319, 332, 1984) hergestellt werden, bei der 1,1-Diaryl-2-halogenethylene umgelagert werden zu Diarylacetylenen in Gegenwart starker Basen.

Tolane der Formel I können auch hergestellt werden, indem man die entsprechenden Stilbene bromiert und anschließend einer Dehydrohalogenierung unterwirft. Dabei kann man an sich bekannte, hier nicht näher erwähnte Varianten dieser Umsetzung anwenden.

Zur Herstellung von Nitrilen der Formel I können entsprechende Säureamide, z.B. solche, in denen an Stelle des Restes CN eine CONH₂-Gruppe steht, dehydratisiert werden. Die Amide sind z.B. aus entsprechenden Estern oder Säurehalogeniden durch Umsetzung mit Ammoniak erhältlich. Als wasserabspaltende Mittel eignen sich beispielsweise anorganische Säurechloride wie SOCl₂, PCl₃, PCl₅, POCl₃, SO₂Cl₂, COCl₂, ferner P₂O₅, P₂S₅, AlCl₃ (z.B. als Doppelverbindung mit NaCl), aromatische Sulfonsäuren und Sulfonsäurehalogenide. Man kann dabei in Gegenwart oder Abwesenheit eines inerten Lösungsmittels bei Temperaturen zwischen etwa 0° und 150° arbeiten; als Lösungsmittel kommen z.B. Basen wie Pyridin oder Triethylamin, aromatische Kohlenwasserstoffe wie Benzol, Toluol oder Xylol oder Amide wie DMF in Betracht.

Zur Herstellung der vorstehend genannten Nitrile der Formel I kann man auch entsprechende Säurehalogenide, vorzugsweise die Chloride, mit Sulfamid umsetzen, zweckmäßig in einem inerten Lösungsmittel wie z. B. Tetramethylensulfon bei Temperaturen zwischen etwa 80° und 150°, vorzugsweise bei 120°. Nach üblicher Aufarbeitung kann man direkt die Nitrile isolieren.

Ether der Formel I sind durch Veretherung entsprechender Hydroxyverbindungen, vorzugsweise entsprechender Phenole, erhältlich, wobei die Hydroxyverbindung zweckmäßig zunächst in ein entsprechendes Metallderivat, z.B. durch Behandeln mit NaH, NaNH₂, NaOH, KOH, Na₂CO₃ oder K₂CO₃ in das entsprechende Alkalimetallalkoholat oder Alkalimetallphenolat übergeführt wird. Dieses kann dann mit dem entsprechenden Alkylhalogenid, -sulfonat oder Dialkylsulfat umgesetzt werden, zweckmäßig in einem inerten Lösungsmittel wie z. B. Aceton, 1,2-Dimethoxyethan, DMF oder Dimethylsulfoxid oder auch mit einem Überschuß an wäßriger oder wäßrig-alkoholischer NaOH oder KOH bei Temperaturen zwischen etwa 20° und 100°.

Zur Herstellung von Nitrilen der Formel I können auch entsprechende Chlor-, Brom- oder Jodverbindungen der Formel I mit einem Cyanid umgesetzt werden, vorzugsweise mit einem Metallcyanid wie z. B. NaCN, KCN oder Cu₂(CN)₂, z.B. in Gegenwart von Pyridin in einem inerten Lösungsmittel wie z.B. DMF oder N-Methylpyrrolidon bei Temperaturen zwischen 20° und 200°.

Verbindungen der Formel I, worin A¹ durch mindestens ein F-Atom und/oder eine CN-Gruppe substituiert ist, können auch aus den entsprechenden Diazoniumsalzen durch Austausch der Diazoniumgruppe gegen ein Fluoratom oder gegen eine CN-Gruppe, z.B. nach den Methoden von Schiemann oder Sandmeyer, erhalten werden.

Dioxanderivate bzw. Dithianderivate der Formel I werden zwecksmäßig durch Reaktion eines entsprechenden Aldehyds (oder eines seiner reaktionsfähigen Derivate) mit einem entsprechenden 1,3-Diol (oder einem seiner reaktionsfähigen Derivate) bzw. einem entsprechenden 1,3-Dithiol hergestellt, vorzugsweise in Gegenwart eines inerten Lösungsmittels wie z.B. Benzol oder Toluol und/oder eines Katalysators, z.B. einer starken Säure wie Schwefelsäure, Benzol- oder p-Toluolsulfonsäure, bei Temperaturen zwischen etwa 20° und etwa 150°, vorzugsweise zwischen 80° und 120°. Als reaktionsfähige Derivate der Ausgangsstoffe eignen sich in erster Linie Acetale.

Die genannten Aldehyde und 1,3-Diole bzw. 1,3-Dithiole sowie ihre reaktionsfähigen Derivate sind zum Teil bekannt, zum Teil können sie ohne Schwierigkeiten nach Standardverfahren der Organischen Chemie aus literaturbekannten Verbindungen hergestellt werden. Beispielsweise sind die Aldehyde durch Oxydation entsprechender Alkohole oder durch Reduktion von Nitrilen oder entsprechenden Carbonsäuren oder ihrer Derivate, die Diole durch Reduktion entsprechender Diester und die Dithiole durch Umsetzung entsprechender Dihalogenide mit NaSH erhältlich.

Die erfindungsgemäßen flüssigkristallinen Medien enthalten vorzugsweise neben einer oder mehreren erfindungsgemäßen Verbindungen als weitere Bestandteile 2 bis 40, insbesondere 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, Phenyl- oder Cyclohexylbenzoate, Cyclohexan-carbonsäurephenyl- oder cyclohexyl-ester, Phenyl- oder Cyclohexyl-ester der Cyclohexylbenzoesäure, Phenyl- oder Cyclohexyl-ester der Cyclohexylcyclohexancarbonsäure, Cyclohexylphenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexene, Cyclohexylcyclohexylcyclohexene, 1,4-Bis-cyclohexylbenzole, 4,4'-Bis-cyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenyl-cyclohexyl)-ethane, 1-Cyclohexyl-2-biphenylylethane, 1-Phenyl-2-cyclohexylphenylethane, gegebenenfalls halogenierten Stilbene, Benzylphenylether, Tolane und substituierten Zimtsäuren. Die 1,4-Phenylengruppen in diesen Verbindungen können auch fluoriert sein.

Die wichtigsten als weitere Bestandteile erfindungsgemäßer Medien in Frage kommenden Verbindungen lassen sich durch die Formeln 1, 2, 3, 4 und 5 charakterisieren:

R'-L-E-R" 1

R'-L-COO-E-R" 2

R'-L-OOC-E-R" 3

R'-L-CH₂CH₂-E-R" 4

R'-L-C≡-C-E-R" 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc, -Pyr-, -Dio-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten.

Vorzugsweise ist einer der Reste L und E Cyc, Phe oder Pyr. E ist vorzugsweise Cyc, Phe oder Phe-Cyc. Vorzugsweise enthalten die erfindinngsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc, -G-Phe- und -G-Cyc-.

R' und R'' bedeuten in den Verbindungen der Teilformeln 1a, 2a, 3a, 4a und 5a jeweils unabhängig voneinander Alkyl, Alkenyl, Alkoxy, Alkenyloxy oder Alkanoyloxy mit bis zu 8 Kohlenstoffatomen. Bei den meisten dieser Verbindungen sind R' und R'' voneinander verschieden, wobei einer dieser Reste meist Alkyl oder Alkenyl ist. In den Verbindungen der Teilformeln 1b, 2b, 3b, 4b und 5b bedeutet R'' -CN, -CF₃, F, Cl oder -NCS; R hat dabei die bei den Verbindungen der Teilformeln 1a bis 5a angegebene Bedeutung und ist vorzugsweise Alkyl oder Alkenyl. Aber auch andere Varianten der vorgesehenen Substituenten in den Verbindungen der Formeln 1, 2, 3, 4 und 5 sind gebräuchlich. Viele solcher Substanzen oder auch Gemische davon sind im Handel erhältlich. Alle diese Substanzen sind nach literaturbekannten Methoden oder in Analogie dazu erhältlich.

Die erfindungsgemäßen Medien enthalten vorzugsweise neben Komponenten aus der Gruppe der Verbindungen 1a, 2a, 3a, 4a und 5a (Gruppe 1) auch Komponenten aus der Gruppe der Verbindungen 1b, 2b, 3b, 4b und 5b (Gruppe 2), deren Anteile vorzugsweise wie folgt sind:
Gruppe 1: 20 bis 90 %, insbesondere 30 bis 90 %,
Gruppe 2: 10 bis 80 %, insbesondere 10 bis 50 %,
wobei die Summe der Anteile der erfindungsgemäßen Verbindungen und der Verbindungen aus den Gruppen 1 und 2 bis zu 100 % ergeben.

Die erfindungsgemäßen Medien enthalten vorzugsweise 1 bis 40 %, insbesondere vorzugsweise 5 bis 30 % an erfindungsgemäßen Verbindungen. Weiterhin bevorzugt sind Medien, enthaltend mehr als 40 %, insbesondere 45 bis 90 % an erfindungegemäßen Verbindungen. Die Medien enthalten vorzugsweise drei, vier oder fünf erfindungsgemäße Verbindungen.

Die Herstellung der erfindungsgemäßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Die folgenden Beispiele sollen die Erfindung erläutern, ohne sie zu begrenzen. Vor- und nachstehend bedeuten Prozentangaben Gewichtsprozent. Alle Temperaturen sind in Grad Celsius angegeben. Fp. bedeutet Schmelzpunkt Kp = Klärpunkt. Ferner bedeuten K = kristalliner Zustand, N = nematische Phase, S = smektische Phase und I = isotrope Phase. Die Angaben zwischen diesen Symbolen stellen die Übergangstemperaturen dar. Δn bedeutet optische Anisotropie (589 nm, 20 °C) und die Viskosität (mm²/sec) wurde bei 20 °C bestimmt.

"Übliche Aufarbeitung" bedeutet: man gibt gegebenenfalls Wasser hinzu, extrahiert mit Methylenchlorid, Diethylether oder Toluol, trennt ab, trocknet die organische Phase, dampft ein und reinigt das Produkt durch Destillation unter reduziertem Druck oder Kristallisation und/oder Chromatographie. Folgende Abkürzungen werden verwendet:
- DAST: Diethylaminoschwefeltrifluorid
- DCC: Dicyclohexylcarbodiimid
- DDQ: Dichlordicyanobenzochinon
- DIBALH: Diisobutylaluminiumhydrid
- KOT: Kalium-tertiär-butanolat
- THF: Tetrahydrofuran
- pTSOH: p-Toluolsulfonsäure

### Beispiel 1

### 4-Difluormethoxy-4'-octoxy-biphenyl

Ein Gemisch aus 20 g 4-Hydroxy-4'-octyloxy-biphenyl, 13,6 g NaOH, 100 ml Wasser und 150 ml Dioxan wird unter Rühren auf 70 °C erhitzt. In die abgekühlte zweiphasige Mischung wird unter heftigem Rühren 10 g Chlordifluormethan eingeleitet. Das Reaktionsgemisch wird auf Wasser gegossen und mit Petrolether extrahiert. Die organische Phase wird über Na₂SO₄ getrocknet, eingedampft und der Rückstand über eine kurze Kieselgelsäule mit Petrolether als Laufmittel filtriert. Das Produkt wird aus Acetonitril umkristallisiert. Man erhält farblose Kristalle. Fₚ: 104 °C, kp: 20 °C (extrapoliert), Δn = 0.093, Viskosität: 17
Analog werden hergestellt:
4-Difluormethoxy-4'-methoxy-biphenyl
4-Difluormethoxy-4'-ethoxy-biphenyl
4-Difluormethoxy-4'-propoxy-biphenyl
4-Difluormethoxy-4'-butoxy-biphenyl, Fp: 122 °C, Δn = 0,146
4-Difluormethoxy-4'-pentoxy-biphenyl
4-Difluormethoxy-4'-hexoxy-biphenyl
4-Difluormethoxy-4'-heptoxy-biphenyl
4-Difluormethoxy-4'-nonoxy-biphenyl
4-Difluormethoxy-4'-decoxy-biphenyl
4-Difluormethoxy-4'-methyl-biphenyl
4-Difluormethoxy-4'-ethyl-biphenyl
1-(4-Difluormethoxyphenyl)-4-ethyl-bicyclo[2.2.2]octan
4-Difluormethoxy-4'-propyl-biphenyl, Fp: 84 °C, Kp (extrapoliert): -30 °C Viskosität: 6
4-Difluormethoxy-4'-butyl-biphenyl
4-Difluormethoxy-4'-pentyl-biphenyl
4-Difluormethoxy-4'-hexyl-biphenyl
4-Difluormethoxy-4'-heptyl-biphenyl
4-Difluormethoxy-4'-nonyl-biphenyl
4-Difluormethoxy-4'-decyl-biphenyl
1-Propyl-3-(4-difluormethoxyphenyl-4'-yl)-cyclobutan
6-(4-Difluormethoxyphenyl)-2-methyl-naphthalin
6-(4-Difluormethoxyphenyl)-2-methyl-1,2,3,4-tetrahydronaphthalin
Difluormethoxy-4-(trans-4-ethylcyclohexyl)-benzol
Difluormethoxy-4-(trans-4-propylcyclohexyl)-benzol
Fₚ: -15 °C, kp: -40 °C (extrapoliert), Δn = 0.035, Viskosität: 5;
Difluormethoxy-4-(trans-4-butylyclohexyl)-benzol
Fp: 8 °C, Kp: -30 °C (extrapoliert), Δn = 0,043, Viskosität: 6;
Difluormethoxy-4-(trans-4-pentylcyclohexyl)-benzol Fp: 1 °C, Kp: -17 °C, Δn = 0.058, Viskosität: 7;
Difluormethoxy-4-(trans-4-hexylcyclohexyl)-benzol
Difluormethoxy-4-(trans-4-heptylcyclohexyl)-benzol
Difluormethoxy-4-(trans-4-octylcyclohexyl)-benzol
Difluormethoxy-4-(trans-4-nonylcyclohexyl)-benzol
Difluormethoxy-4-(trans-4-decylcyclohexyl)-benzol
4-Difluormethoxy-2',3'-difluor-4'-methoxy-biphenyl
4-Difluormethoxy-2',3'-difluor-4'-ethoxy-biphenyl
4-Difluormethoxy-2',3'-difluor-4'-propoxy-biphenyl
4-Difluormethoxy-2',3'-difluor-4'-butoxy-biphenyl
4-Difluormethoxy-2',3'-difluor-4'-pentoxy-biphenyl
4-Difluormethoxy-2',3'-difluor-4'-hexoxy-biphenyl
4-Difluormethoxy-2',3'-difluor-4'-heptoxy-biphenyl
4-Difluormethoxy-2',3'-difluor-4'-octoxy-biphenyl
4-Difluormethoxy-2',3'-difluor-4'-nonoxy-biphenyl
4-Difluormethoxy-2',3'-difluor-4'-decoxy-biphenyl
4-Difluormethoxy-2',3'-difluor-4'-methyl-biphenyl
4-Difluormethoxy-2',3'-difluor-4'-ethyl-biphenyl
4-Difluormethoxy-2',3'-difluor-4'-propyl-biphenyl
4-Difluormethoxy-2',3'-difluor-4'-butyl-biphenyl
4-Difluormethoxy-2',3'-difluor-4'-pentyl-biphenyl
4-Difluormethoxy-2',3'-difluor-4'-hexyl-biphenyl
4-Difluormethoxy-2',3'-difluor-4'-heptyl-biphenyl
4-Difluormethoxy-2',3'-difluor-4'-octyl-biphenyl
4-Difluormethoxy-2',3'-difluor-4'-nonyl-biphenyl
4-Difluormethoxy-2',3'-difluor-4'-decyl-biphenyl
4-Difluormethoxy-4'-cyano-3'-fluorbiphenyl
4-Difluormethoxy-4'-methyl-terphenyl
4-Difluormethoxy-4'-ethyl-terphenyl
4-Difluormethoxy-4'-propyl-terphenyl
4-Difluormethoxy-4'-butyl-terphenyl
4-Difluormethoxy-4'-pentyl-terphenyl, Fp: 223 °C, Kp: 241 °C
4-Difluormethoxy-4'-hexyl-terphenyl
4-Difluormethoxy-4'-heptyl-terphenyl
4-Difluormethoxy-4'-octyl-terphenyl
4-Difluormethoxy-4'-nonyl-terphenyl
4-Difluormethoxy-4'-decyl-terphenyl
4-Difluormethoxy-4'-(trans-4-methylcyclohexyl)-biphenyl
4-Difluormethoxy-4'-(trans-4-ethylcyclohexyl)-biphenyl
4-Difluormethoxy-4'-(trans-4-propylcyclohexyl)-biphenyl, Fp: 82 °C, Kp: 169,4 °C, Δn = 0,174
4-Difluormethoxy-4'-(trans-4-butylcyclohexyl)-biphenyl
4-Difluormethoxy-4'-(trans-4-pentylcyclohexyl)-biphenyl, Fp: 67 °C, Kp: 161,8 °C, Δn = 0,115
4-Difluormethoxy-4'-(trans-4-hexylcyclohexyl)-biphenyl
4-Difluormethoxy-4'-(trans-4-heptylcyclohexyl)-biphenyl
4-Difluormethoxy-4'-(trans-4-otylcyclohexyl)-biphenyl
4-Difluormethoxy-4'-(trans-4-nonylcyclohexyl)-biphenyl
4-Difluormethoxy-4'-(trans-4-decylcyclohexyl)-biphenyl
4-Methyl-4'-(4-difluormethoxyphenyl)-trans,trans-bicyclohexyl
4-Trifluormethyl-4'-(4-difluormethoxyphenyl)-trans,trans-bicyclohexyl
4-Ethyl-4'-(4-difluormethoxyphenyl)-trans,trans-bicyclohexyl
4-Propyl-4'-(4-difluormethoxyphenyl)-trans,trans-bicyclohexyl, Fp: 39 °C, Kp: 148,6 °C, Δn = 0,088, Viskosität: 16
4-Isopropyl-4'-(4-difluormethoxyphenyl)-trans,trans-bicyclohexyl
4-Butyl-4'-(4-difluormethoxyphenyl)-trans,trans-bicyclohexyl
4-Methoxyethyl-4'-(4-difluormethoxyphenyl)-trans,trans-bicyclohexyl
4-Pentyl-4'-(4-difluormethoxynhenyl)-trans,trans-bicyclohexyl
4-Hexyl-4'-(4-difluormethoxyphenyl)-trans,trans-bicyclohexyl
4-Heptyl-4'-(4-difluormethoxyphenyl)-trans,trans-bicyclohexyl
4-Octyl-4'-(4-difluormethoxyphenyl)-trans,trans-bicyclohexyl
4-Nonyl-4'-(4-difluormethoxyphenyl)-trans,trans-bicyclohexyl
4-Decyl-4'-(4-difluormethoxyphenyl)-trans,trans-bicyclohexyl
1-(4-Difluormethoxyphenyl)-4-(trans-4-methylcyclohexyl)-cyclohexen
1-(4-Difluormethoxyphenyl)-4-(trans-4-ethylcyclohexyl)-cyclohexen
1-(4-Difluormethoxyphenyl)-4-(trans-4-propylcyclohexyl)-cyclohexen
1-(4-Difluormethoxyphenyl)-4-(trans-4-butylcyclohexyl)-cyclohexen
1-(4-Difluormethoxyphenyl)-4-(trans-4-pentylcyclohexyl)-cyclohexen
1-(4-Difluormethoxyphenyl)-4-(trans-4-hexylcyclohexyl)-cyclohexen
1-(4-Difluormethoxyphenyl)-4-(trans-4-heptylcyclohexyl)-cyclohexen
1-(4-Difluormethoxyphenyl)-4-(trans-4-octylcyclohexyl)-cyclohexen
1-(4-Difluormethoxyphenyl)-4-(trans-4-nonylcyclohexyl)-cyclohexen
1-(4-Difluormethoxyphenyl)-4-(trans-4-decylcyclohexyl)-cyclohexen
1-(4-Difluormethoxyphenyl)-trans-4-(1-methylcyclohexen-4-yl)-cyclohexan
1-(4-Difluormethoxyphenyl)-trans-4-(1-ethylcyclohexen-4-yl)-cyclohexan
1-(4-Difluormethoxyphenyl)-trans-4-(1-propylcyclohexen-4-yl)-cyclohexan
1-(4-Difluormethoxyphenyl)-trans-4-(1-butylcyclohexen-4-yl)-cyclohexan
1-(4-Difluormethoxyphenyl)-trans-4-(1-pentylcyclohexen-4-yl)-cyclohexan
1-(4-Difluormethoxyphenyl)-trans-4-(1-hexylcyclohexen-4-yl)-cyclohexan
1-(4-Difluormethoxyphenyl)-trans-4-(1-heptylcyclohexen-4-yl)-cyclohexan
1-(4-Difluormethoxyphenyl)-trans-4-(1-octylcyclohexen4-yl)-cyclohexan
1-(4-Difluormethoxyphenyl)-trans-4-(1-nonylcyclohexen4-yl)-cyclohexan
1-(4-Difluormethoxyphenyl)-trans-4-(1-decylcyclohexen-4-yl)-cyclohexan
4-Methyl-4'-(3-fluor-4-difluormethoxyphenyl)-trans,trans-bicyclohexyl
4-Ethyl-4'-(3-fluor-4-difluormethoxyphenyl)-trans,trans-bicyclohexyl
4-Propyl-4'-(3,5-difluor-4-difluormethoxyphenyl)-trans,trans-bicyclohexyl
4-Propyl-4'-(3-fluor-4-difluormethoxyphenyl)-trans,trans-bicyclohexyl, Fp: 33 °C, Kp: 144 °C, Δn = 0,106
4-Butyl-4'-(3-fluor-4-difluormethoxyphenyl)-trans,trans-bicyclohexyl
4-Pentyl-4'-(3-fluor-4-difluormethoxyphenyl)-trans,trans-bicyclohexyl
4-Hexyl-4'-(3-fluor-4-difluormethoxyphenyl)-trans,trans-bicyclohexyl
4-Heptyl-4'-(3-fluor-4-difluormethoxyphenyl)-trans,trans-bicyclohexyl
4-Octyl-4'-(3-fluor-4-difluormethoxyphenyl)-trans,trans-bicyclohexyl
4-Nonyl-4'-(3-fluor-4-difluormethoxyphenyl)-trans,trans-bicyclohexyl
4-Decyl-4'-(3-fluor-4-difluormethoxyphenyl)-trans,trans-bicyclohexyl
Aus entsprechenden Thiolen werden analog hergestellt:
4-Difluormethylthio-4'-methyl-biphenyl
4-Difluormethylthio-4'-ethyl-biphenyl
4-Difluormethylthio-4'-propyl-biphenyl
4-Difluormethylthio-4'-butyl-biphenyl
4-Difluormethylthio-4'-pentyl-biphenyl
4-Difluormethylthio-4'-hexyl-biphenyl
4-Difluormethylthio-4'-heptyl-biphenyl
4-Difluormethylthio-4'-octyl-biphenyl
4-Difluormethylthio-4'-nonyl-biphenyl
4-Difluormethylthio-4'-decyl-biphepyl
Difluormethylthio-4-(trans-4-methylcyclohexyl)-benzol
Difluormethylthio-4-(trans-4-ethylcyclohexyl)-benzol
Difluormethylthio-4-(trans-4-propylcyclohexyl)-benzol
Difluormethylthio-4-(trans-4-butylcyclohexyl)-benzol
Difluormethylthio-4-(trans-4-pentylcyclohexyl)-benzol
Difluormethylthio-4-(trans-4-hexylcyclohexyl)-benzol
Difluormethylthio-4-(trans-4-heptylcyclohexyl)-benzol
Difluormethylthio-4-(trans-4-octylcyclohexyl)-benzol
Difluormethylthio-4-(trans-4-nonylcyclohexyl)-benzol
Difluormethylthio-4-(trans-4-decylcyclohexyl)-benzol
4-Difluormethylthio-4'-(trans-4-methylcyclohexyl)-biphenyl
4-Difluormethylthio-4'-(trans-4-ethylcyclohexyl)-biphenyl
4-Difluormethylthio-4'-(trans-4-propylcyclohexyl)-biphenyl
4-Difluormethylthio-4'-(trans-4-butylcyclohexyl)-biphenyl
4-Difluormethylthio-4'-(trans-4-pentylcyclohexyl)-biphenyl
4-Difluormethylthio-4'-(trans-4-hexylcyclohexyl)-biphenyl
4-Difluormethylthio-4'-(trans-4-heptylcyclohexyl)-biphenyl
4-Difluormethylthio-4'-(trans-4-octylcyclohexyl)-biphenyl
4-Difluormethylthio-4'-(trans-4-nonylcyclohexyl)-biphenyl
4-Difluormethylthio-4'-(trans-4-decylcyclohexyl)-biphenyl

### Beispiel 2

### Difluormethoxy-(5-propyl-1.3-dioxan-2-yl)-benzol

17,2 g p-Difluormethoxybenzaldehyd (kommerziell bei Fluorochem. Ltd. (GB) erhältlich), 10,4 g Ethylpropandiol und 0.2 g p-TsOH werden in 100 ml Toluol 2 h zum Sieden erhitzt. Nach Eindampfen des Lösungsmittels wird wie üblich aufgearbeitet.

Analog werden hergestellt:
Difluormethoxy-(5-ethyl-1,3-dioxan-2-yl)-benzol
Difluormethoxy-(5-butyl-1,3-dioxan-2-yl)-benzol
Difluormethoxy-(5-pentyl-1,3-dioxan-2-yl)-benzol
Difluormethoxy-(5-hexyl-1,3-dioxan-2-yl)-benzol
Difluormethoxy-(5-heptyl-1,3-dioxan-2-yl)-benzol
Difluormethoxy-(5-octyl-1,3-dioxan-2-yl)-benzol
Difluormethoxy-(5-nonyl-1,3-dioxan-2-yl)-benzol
Difluormethoxy-(5-decyl-1,3-dioxan-2-yl)-benzol
Difluormethoxy-4-[5-(trans-4-methylcyclohexyl)-1,3-dioxan-2-yl]-benzol
Difluormethoxy-4-[5-(trans-4-ethylcyclohexyl)-1,3-dioxan-2-yl]-benzol
Difluormethoxy-4-[5-(trans-4-propylcyclohexyl)-1,3-dioxan-2-yl]-benzol
Difluormethoxy-4-[5-(trans-4-butylcyclohexyl)-1,3-dioxan-2-yl]-benzol
Difluormethoxy-4-[5-(trans-4-pentylcyclohexyl)-1,3-dioxan-2-yl]-benzol
Difluormethoxy-4-[5-(trans-4-hexylcyclohexyl)-1,3-dioxan-2-yl]-benzol
Difluormethoxy-4-[5-(trans-4-heptylcyclohexyl)-1,3-dioxan-2-yl]-benzol
Difluormethoxy-4-[5-(trans-4-octylcyclohexyl)-1,3-dioxan-2-yl]-benzol
Difluormethoxy-4-[5-(trans-4-nonylcyclohexyl)-1,3-dioxan-2-yl]-benzol
Difluormethoxy-4-[5-(trans-4-decylcyclohexyl)-1,3-dioxan-2-yl]-benzol

### Beispiel 3

### Difluormethoxy-4-(5-heptyl-1,3-pyrimidin-2-yl)-benzol

Ein Gemisch aus 22,2 g p-Difluormethoxy-benzimidamidhydrochlorid (erhältlich aus dem Nitril über das entsprechende Benzimidsäureethylesterhydrochlorid) und 31,8 g Heptylmalondialdehydbisdiethylacetal wird 15 h bei 150 °C gerührt. Nach dem Abkühlen wird wie üblich aufgearbeitet. Fp: 26 °C, Kp: 32 °C, Δn: 0,112, Viskosität: 16.

Analog werden hergestellt:
Difluormethoxy-4-(5-methyl-1,3-pyrimidin-2-yl)-benzol
Difluormethoxy-4-(5-ethyl-1,3-pyrimidin-2-yl)-benzol
Difluormethoxy-4-(5-propyl-1,3-pyrimidin-2-yl)-benzol, Fp: 41 °C, Kp (extrapoliert): 0 °C, Δn = 0,150, Viskosität: 14.
Difluormethoxy-4-(5-butyl-1,3-pyrimidin-2-yl)-benzol
Difluormethoxy-4-(5-pentyl-1,3-pyrimidin-2-yl)-benzol, Fp: 21 °C, Kp: 26 °C, Δn: 0,13, Viskosität: 14
Difluormethoxy-4-(5-hexyl-1,3-pyrimidin-2-yl)-benzol
Difluormethoxy-4-(5-octyl-1,3-pyrimidin-2-yl)-benzol
Difluormethoxy-4-(5-nonyl-1,3-pyrimidin-2-yl)-benzol
Difluormethoxy-4-(5-decyl-1,3-pyrimidin-2-yl)-benzol
Difluormethoxy-4-(5-methoxy-1,3-pyrimidin-2-yl)-benzol
Difluormethoxy-4-(5-ethoxy-1,3-pyrimidin-2-yl)-benzol
Difluormethoxy-4-(5-propoxy-1,3-pyrimidin-2-yl)-benzol
Difluormethoxy-4-(5-butoxy-1,3-pyrimidin-2-yl)-benzol
Difluormethoxy-4-(5-pentoxy-1,3-pyrimidin-2-yl)-benzol
Difluormethoxy-4-(5-hexoxy-1,3-pyrimidin-2-yl)-benzol
Difluormethoxy-4-(5-heptoxy-1,3-pyrimidin-2-yl)-benzol
Difluormethoxy-4-(5-octoxy-1,3-pyrimidin-2-yl)-benzol
Difluormethoxy-4-(5-nonoxy-1,3-pyrimidin-2-yl)-benzol
Difluormethoxy-4-(5-decoxy-1,3-pyrimidin-2-yl)-benzol
Difluormethoxy-4-[5-(trans-4-methylcyclohexyl)-1,3-pyrimidin-2-yl]-benzol
Difluormethoxy-4-[5-(trans-4-ethylcyclohexyl)-1,3-pyrimidin-2-yl]-benzol
Difluormethoxy-4-[5-(trans-4-propylcyclohexyl)-1,3-pyrimidin-2-yl]-benzol
Difluormethoxy-4-[5-(trans-4-butylcyclohexyl)-1,3-pyrimidin-2-yl]-benzol
Difluormethoxy-4-[5-(trans-4-pentylcyclohexyl)-1,3-pyrimidin-2-yl]-benzol
Difluormethoxy-4-[5-(trans-4-hexylcyclohexyl)-1,3-pyrimidin-2-yl]-benzol
Difluormethoxy-4-[5-(trans-4-heptylcyclohexyl)-1,3-pyrimidin-2-yl]-benzol
Difluormethoxy-4-[5-(trans-4-octylcyclohexyl)-1,3-pyrimidin-2-yl]-benzol
Difluormethoxy-4-[5-(trans-4-nonylcyclohexyl)-1,3-pyrimidin-2-yl]-benzol
Difluormethoxy-4-[5-(trans-4-decylcyclohexyl)-1,3-pyrimidin-2-yl]-benzol
Difluormethoxy-4-[5-(2-(trans-4-methylcyclohexyl)-ethyl)-1,3-pyrimidin-2-yl]-benzol
Difluormethoxy-4-[5-(2-(trans-4-ethylcyclohexyl)-ethyl)-1,3-pyrimidin-2-yl]-benzol
Difluormethoxy-4-[5-(2-(trans-4-propylcyclohexyl)-ethyl)-1,3-pyrimidin-2-yl]-benzol
Difluormethoxy-4-[5-(2-(trans-4-butylcyclohexyl)-ethyl)-1,3-pyrimidin-2-yl]-benzol
Difluormethoxy-4-[5-(2-(trans-4-pentylcyclohexyl)-ethyl)-1,3-pyrimidin-2-yl]-benzol
Difluormethoxy-4-[5-(2-(trans-4-hexylcyclohexyl)-ethyl)-1,3-pyrimidin-2-yl]-benzol
Difluormethoxy-4-[5-(2-(trans-4-heptylcyclohexyl)-ethyl)-1,3-pyrimidin-2-yl]-benzol
Difluormethoxy-4-[5-(2-(trans-4-octylcyclohexyl)-ethyl)-1,3-pyrimidin-2-yl]-benzol
Difluormethoxy-4-[5-(2-(trans-4-nonylcyclohexyl)-ethyl)-1,3-pyrimidin-2-yl]-benzol
Difluormethoxy-4-[5-(2-(trans-4-decylcyclohexyl)-ethyl)-1,3-pyrimidin-2-yl]-benzol
Mit 4-Mercaptobenzonitril (erhältlich aus 4-Cyanophenol durch Umsetzung mit Dimethylcarbamoylchlorid und thermische Umlagerung) werden analog dargestellt:
Difluormethylthio-4-(5-methyl-1,3-pyrimidin-2-yl)-benzol
Difluormethylthio-4-(5-ethyl-1,3-pyrimidin-2-yl)-benzol
Difluormethylthio-4-(5-propyl-1,3-pyrimidin-2-yl)-benzol
Difluormethylthio-4-(5-butyl-1,3-pyrimidin-2-yl)-benzol
Difluormethylthio-4-(5-pentyl-1,3-pyrimidin-2-yl)-benzol
Difluormethylthio-4-(5-hexyl-1,3-pyrimidin-2-yl)-benzol
Difluormethylthio-4-(5-heptyl-1,3-pyrimidin-2-yl)-benzol
Difluormethylthio-4-(5-octyl-1,3-pyrimidin-2-yl)-benzol
Difluormethylthio-4-(5-nonyl-1,3-pyrimidin-2-yl)-benzol
Difluormethylthio-4-(5-decyl-1,3-pyrimldin-2-yl)-benzol
Difluormethylthio-4-(5-methoxy-1,3-pyrimidin-2-yl)-benzol
Difluormethylthio-4-(5-ethoxy-1,3-pyrimidin-2-yl)-benzol
Difluormethylthio-4-(5-propoxy-1,3-pyrimidin-2-yl)-benzol
Difluormethylthio-4-(5-butoxy-1,3-pyrimidin-2-yl)-benzol
Difluormethylthio-4-(5-pentoxy-1,3-pyrimidin-2-yl)-benzol
Difluormethylthio-4-(5-hexoxy-1,3-pyrimidin-2-yl)-benzol
Difluormethylthio-4-(5-heptoxy-1,3-pyrimidin-2-yl)-benzol
Difluormethylthio-4-(5-octoxy-1,3-pyrimidin-2-yl)-benzol
Difluormethylthio-4-(5-nonoxy-1,3-pyrimidin-2-yl)-benzol
Difluormethylthio-4-(5-decoxy-1,3-pyrimidin-2-yl)-benzol
Difluormethoxy-4-(5-methylpyridin-2-yl)-benzol
Difluormethoxy-4-(5-ethylpyridin-2-yl)-benzol
Difluormethoxy-4-(5-propylpyridin-2-yl)-benzol
Difluormethoxy-4-(5-butylpyridin-2-yl)-benzol
Difluormethoxy-4-(5-pentylpyridin-2-yl)-benzol
Difluormethoxy-4-(5-hexylpyridin-2-yl)-benzol
Difluormethoxy-4-(5-heptylpyridin-2-yl)-benzol
Difluormethoxy-4-(5-octylpyridin-2-yl)-benzol
Difluormethoxy-2-fluor-4-(2-octylpyridin-5-yl)-benzol
Difluormethoxy-4-(5-nonylpyridin-2-yl)-benzol
Difluormethoxy-4-(5-decylpyridin-2-yl)-benzol
Difluormethoxy-4-(5-methoxypyridin-2-yl)-benzol
Difluormethoxy-4-(5-ethoxypyridin-2-yl)-benzol
Difluormethoxy-4-(5-propoxypyridin-2-yl)-benzol
Difluormethoxy-4-(5-butoxypyridin-2-yl)-benzol
Difluormethoxy-4-(5-pentoxypyridin-2-yl)-benzol
Difluormethoxy-4-(5-hexoxypyridin-2-yl)-benzol
Difluormethoxy-4-(5-heptoxypyridin-2-yl)-benzol
Difluormethoxy-4-(5-octoxypyridin-2-yl)-benzol
Difluormethoxy-2-fluor-4-(2-octoxypyridin-5-yl)-benzol
Difluormethoxy-4-(5-nonoxypyridin-2-yl)-benzol
Difluormethoxy-4-(5-decoxypyridin-2-yl)-benzol
4-Difluormethoxy-4'-(5-methyl-1,3-pyrimidin-2-yl)-biphenyl
4-Difluormethoxy-4'-(5-ethyl-1,3-pyrimidin-2-yl)-biphenyl
4-Difluormethoxy-4'-(5-propyl-1,3-pyrimidin-2-yl)-biphenyl
4-Difluormethoxy-4'-(5-butyl-1,3-pyrimidin-2-yl)-biphenyl
4-Difluormethoxy-4'-(5-pentyl-1,3-pyrimidin-2-yl)-biphenyl
4-Difluormethoxy-4'-(5-hexyl-1,3-pyrimidin-2-yl)-biphenyl
4-Difluormethoxy-4'-(5-heptyl-1,3-pyrimidin-2-yl)-biphenyl
4-Difluormethoxy-4'-(5-octyl-1,3-pyrimidin-2-yl)-biphenyl
4-Difluormethoxy-4'-(5-nonyl-1,3-pyrimidin-2-yl)-biphenyl
4-Difluormethoxy-4'-(5-decyl-1,3-pyrimidin-2-yl)-biphenyl

### Beispiel 4

### 4-Benzyloxy-difluormethoxy-benzol

Ein Gemisch aus 40,0 g Hydrochinonmonobenzylether, 40 g NaOH, 200 ml Wasser und 300 ml Dioxan wird unter Rühren auf 70 °C erhitzt. In die abgekühlte Mischung wird unter heftigem Rühren 35.5 g Chlordifluormethan eingeleitet.

Das Reaktionsgemisch wird auf Wasser gegossen und mit Petrolether extrahiert. Die organische Phase wird über Na₂SO₄ getrocknet, eingedampft und der Rückstand über eine kurze Kieselgelsäule mit Petrolether/Ethylacetat 8:2 als Laufmittel chromatographiert. Man erhält eine farblose Flüssigkeit.

### Beispiel 5

a) Hydrochinon-mono-difluormethyl-ether
   Eine Lösung von 25,0 g des Produkts aus Beispiel 4 in 100 ml THF wird mit 8 g Pd/C (5 % Pd) als Katalysator bei Raumtemperatur und Normaldruck hydriert. Der Katalysator wird abfiltriert und das Filtrat eingedampft.
b) 4-Difluormethoxy-phenyl-trans-4-pentyl-cyclohexylcarboxylat
   In einer auf 0 °C gehaltenen Lösung von 9,92 g trans-4-Pentyl-cyclohexyl-carbonsäure, 8,11 g Hydrochinonmono-difluormethyl-ether und 611 mg 4-Dimethylaminopyridin in 100 ml Dichlormethan wird eine Lösung von 10,3 g DCC in 50 ml Dichlormethan zugetropft. Nach 18 h Rühren bei Raumtemperatur wird der entstandene Niederschlag abfiltriert und das Filtrat eingedampft. Der Rückstand wird über eine Kieselgelsäule mit Petrolether/Ethylacetat 9:1 als Laufmittel chromatographiert. Man erhält farblose Kristalle, Fp: 54 °C, Kp: 30 °C, Δn = 0,061, Viskosität: 16.

Analog werden hergestellt:
4-Difluormethoxy-phenyl-trans-4-ethyl-cyclohexylcarboxylat
4-Difluormethoxy-phenyl-trans-4-propyl-cyclohexylcarboxylat, Fp: 54°C, Kp: 0 °C, Δn = 0,052, Vikosität: 10
4-Difluormethoxy-phenyl-trans-4-butyl-cyclohexylcarboxylat
4-Difluormethoxy-phenyl-trans-4-hexyl-cyclohexylcarboxylat
4-Difluormethoxy-phenyl-trans-4-heptyl-cyclohexylcarboxylat
4-Difluormethoxy-phenyl-trans-4-octyl-cyclohexylcarboxylat
4-Difluormethoxy-phenyl-trans-4-nonyl-cyclohexylcarboxylat
4-Difluormethoxy-phenyl-trans-4-decyl-cyclohexylcarboxylat
4-Difluormethoxybiphenyl-4'-yl-trans-4-methylcyclohexylcarboxylat
4-Difluormethoxybiphenyl-4'-yl-trans-4-ethylcyclohexylcarboxylat
4-Difluormethoxybiphenyl-4'-yl-trans-4-propylcyclohexylcarboxylat
4-Difluormethoxybiphenyl-4'-yl-trans-4-butylcyclohexylcarboxylat
4-Difluormethoxybiphenyl-4'-yl-trans-4-pentylcyclohexylcarboxylat
4-Difluormethoxybiphenyl-4'-yl-trans-4-hexylcyclohexylcarboxylat
4-Difluormethoxybiphenyl-4'-yl-trans-4-heptylcyclohexylcarboxylat
4-Difluormethoxybiphenyl-4'-yl-trans-4-octylcyclohexylcarboxylat
4-Difluormethoxybiphenyl-4'-yl-trans-4-nonylcyclohexylcarboxylat
4-Difluormethoxybiphenyl-4'-yl-trans-4-decylcyclohexylcarboxylat
4-Difluormethoxy-phenyl-4'-methyl-trans,trans-bicyclohexyl-4-carboxylat
4-Difluormethoxy-phenyl-4'-ethyl-trans,trans-bicyclohexyl-4-carboxylat
4-Difluormethoxy-phenyl-4'-propyl-trans,trans-bicyclohexyl-4-carboxylat
4-Difluormethoxy-phenyl-4'-butyl-trans,trans-bicyclohexyl-4-carboxylat
4-Difluormethoxy-phenyl-4'-pentyl-trans,trans-bicyclohexyl-4-carboxylat, Fp: 61 °C, Kp: 196,9 °C, Δn = 0,089
4-Difluormethoxy-phenyl-4'-hexyl-trans,trans-bicyclohexyl-4-carboxylat
4-Difluormethoxy-phenyl-4'-heptyl-trans,trans-bicyclohexyl-4-carboxylat
4-Difluormethoxy-phenyl-4'-octyl-trans,trans-bicyclohexyl-4-carboxylat
4-Difluormethoxy-phenyl-4'-nonyl-trans,trans-bicyclohexyl-4-carboxylat
4-Difluormethoxy-phenyl-4'-decyl-trans,trans-bicyclohexyl-4-carboxylat
Analog werden hergestellt mit 4-Difluormethylthiophenol (erhältlich durch Veresterung von 4-Hydroxybenzolsulfonsäure (Aldrich) mit Essigsäure, Überführung in das Sulfonylchlorid, Reduktion zum Thiol mit Zink/Salzsäure, Veretherung analog Beispiel 1) und alkalische Etherspaltung):
4-Difluormethylthio-phenyl-trans-4-methyl-cyclohexylcarboxylat
4-Difluormethylthio-phenyl-trans-4-ethyl-cyclohexylcarboxylat
4-Difluormethylthio-phenyl-trans-4-propyl-cyclohexylcarboxylat
4-Difluormethylthio-phenyl-trans-4-butyl-cyclohexylcarboxylat
4-Difluormethylthio-phenyl-trans-4-pentyl-cyclohexylcarboxylat
4-Difluormethylthio-phenyl-trans-4-hexyl-cyclohexylcarboxylat
4-Difluormethylthio-phenyl-trans-4-heptyl-cyclohexylcarboxylat
4-Difluormethylthio-phenyl-trans-4-octyl-cyclohexylcarboxylat
4-Difluormethylthio-phenyl-trans-4-nonyl-cyclohexylcarboxylat
4-Difluormethylthio-phenyl-trans-4-decyl-cyclohexylcarboxylat
3-Fluor-4-difluormethylthio-phenyl-4'-methyl-trans,trans-bicyclohexyl-4-carboxylat
3-Fluor-4-difluormethylthio-phenyl-4'-ethyl-trans,trans-bicyclohexyl-4-carboxylat
3-Fluor-4-difluormethylthio-phenyl-4'-propyl-trans,trans-bicyclohexyl-4-carboxylat
3-Fluor-4-difluormethylthio-phenyl-4'-butyl-trans,trans-bicyclohexyl-4-carboxylat
3-Fluor-4-difluornethylthio-phenyl-4'-pentyl-trans,trans-bicyclohexyl-4-carboxylat
3-Fluor-4-difluormethylthio-phenyl-4'-hexyl-trans,trans-bicyclohexyl-4-carboxylat
3-Fluor-4-difluormethylthio-phenyl-4'-heptyl-trans,trans-bicyclohexyl-4-carboxylat
3-Fluor-4-difluormethylthio-phenyl-4'-octyl-trans,trans-bicyclohexyl-4-carboxylat
3-Fluor-4-difluormethylthio-phenyl-4'-nonyl-trans,trans-bicyclohexyl-4-carboxylat
3-Fluor-4-difluormethylthio-phenyl-4'-decyl-trans,trans-bicyclohexyl-4-carboxylat
2,3-Difluor-4-difluormethylthio-phenyl-trans-4-methyl-cyclohexyl-benzoat
2,3-Difluor-4-difluormethylthio-phenyl-trans-4-ethyl-cyclohexyl-benzoat
2,3-Difluor-4-difluormethylthio-phenyl-trans-4-propyl-cyclohexyl-benzoat
2,3-Difluor-4-difluormethylthio-phenyl-trans-4-butyl-cyclohexyl-benzoat
2,3-Difluor-4-difluormethylthio-phenyl-trans-4-pentyl-cyclohexyl-benzoat
2,3-Difluor-4-difluormethylthio-phenyl-trans-4-hexyl-cyclohexyl-benzoat
2,3-Difluor-4-difluormethylthio-phenyl-trans-4-heptyl-cyclohexyl-benzoat
2,3-Difluor-4-difluormethylthio-phenyl-trans-4-octyl-cyclohexyl-benzoat
2,3-Difluor-4-difluormethylthio-phenyl-trans-4-nonyl-cyclohexyl-benzoat
2,3-Difluor-4-difluormethylthio-phenyl-trans-4-decyl-cyclohexyl-benzoat

### Beispiel 6

a) 4-[2-(4-Benzyloxyphenyl)-ethenyl]-4'-pentyl-trans, trans-bicyclohexyl
   Eine Suspension von 147,1 g 4'-Pentyl-trans,trans-bicyclohexyl-4-methyl-triphenylphosphoniumiodid in 1 l THF wird unter Eiskühlung mit 49,6 g 4-Benzyloxybenzaldehyd und 25,9 g KOT versetzt. Die Mischung wird 1 h bei 5 °C gerührt. Danach wird 2 N HCl bis zur neutralen Reaktion der wäßrigen Phase und Wasser bis zur Lösung des Niederschlags zugegeben. Die organische Phase wird abgetrennt, über Na₂SO₄ getrocknet und eingedampft. Der Rückstand wird über eine Kieselgelsäule mit Petrolether/Ethylacetat 95:5 als Laufmittel chromatographiert. Man erhält farblose Kristalle.
b) 4-[2-(4-Hydroxyphenyl)ethyl]-4'-pentyl-trans,trans-bicyclohexyl
   Eine Lösung von 34,6 g des Produkts 6a in 200 ml THF wird mit 10 g Pd/C (5 % Pd) als Katalysator bei Raumtemperatur und Normaldruck hydriert. Der Katalysator wird abfiltriert und das Filtrat eingedampft. Man erhält einen grauen kristallinen Feststoff;
c) 4-[2(4-Difluormethoxyphenyl)-ethyl]-4'-pentyl-trans, trans-bicyclohexyl
   Das Produkt 6b wird analog Beispiel 1 in die Difluormethoxyverbindung überführt, Fp: 24 °C, Kp: 149,9 °C, Δn = 0,097, Viskosität: 21.
d) Das Phosphoniumiodid aus 6 a) wird direkt mit 4-Difluormethoxybenzaldehyd, wie in 6 a) beschrieben, umgesetzt und analog 6 b) hydriert.

Analog werden hergestellt:
4-[2-(4-Difluormethoxyphenyl)-ethyl]-4'-ethyl-trans, trans-bicyclohexyl
4-[2-(4-Difluormethoxyphenyl)-ethyl]-4'-propyl-trans, trans-bicyclohexyl
4-[2-(4-Difluormethoxyphenyl)-ethyl]-4'-butyl-trans, trans-bicyclohexyl
4-[2-(4-Difluormethoxyphenyl)-ethyl]-4'-hexyl-trans, trans-bicyclohexyl
4-[2-(4-Difluormethoxyphenyl)-ethyl]-4'-heptyl-trans, trans-bicyclohexyl
4-[2-(4-Difluormethoxyphenyl)-ethyl]-4'-octyl-trans, trans-bicyclohexyl
4-[2-(4-Difluormethoxyphenyl)-ethyl]-4'-nonyl-trans, trans-bicyclohexyl
4-[2-(4-Difluormethoxyphenyl)-ethyl]-4'-decyl-trans, trans-bicyclohexyl
4-[2-(2,3-Difluor-4-difluormethoxyphenyl)-ethyl]-4'-methyl-trans,trans-bicyclohexyl
4-[2-(2,3-Difluor-4-difluormethoxyphenyl)-ethyl]-4'-ethyl-trans,trans-bicyclohexyl
4-[2-(2,3-Difluor-4-difluormethoxyphenyl)-ethyl]-4'-propyl-trans,trans-bicyclohexyl, 81 N 128 I
4-[2-(2,3-Difluor-4-difluormethoxyphenyl)-ethyl]-4'-butyl-trans,trans-bicyclohexyl
4-[2-(2,3-Difluor-4-difluormethoxyphenyl)-ethyl]-4'-pentyl-trans,trans-bicyclohexyl
4-[2-(2,3-Difluor-4-difluormethoxyphenyl)-ethyl]-4'-hexyl-trans,trans-bicyclohexyl
4-[2-(2,3-Difluor-4-difluormethoxyphenyl)-ethyl]-4'-heptyl-trans,trans-bicyclohexyl
4-[2-(2,3-Difluor-4-difluormethoxyphenyl)-ethyl]-4'-octyl-trans,trans-bicyclohexyl
4-[2-(2,3-Difluor-4-difluormethoxyphenyl)ethyl]-4'-nonyl-trans,trans-bicyclohexyl
4-[2-(2,3-Difluor-4-difluormethoxyphenyl)-ethyl]-4'-decyl-trans,trans-bicyclohexyl
4-[2-(trans-4-methylcyclohexyl)-ethyl]-4'-difluormethoxybiphenyl
4-[2-(trans-4-ethylcyclohexyl)-ethyl]-4'-difluormethoxybiphenyl
4-[2-(trans-4-propylcyclohexyl)-ethyl]-4'-difluormethoxybiphenyl
4-[2-(trans-4-butylcyclohexyl)-ethyl]-4'-difluormethoxybiphenyl
4-[2-(trans-4-pentylcyclohexyl)-ethyl]-4'-difluormethoxybiphenyl
4-[2-(trans-4-hexylcyclohexyl)-ethyl]-4'-difluormethoxybiphenyl
4-[2-(trans-4-heptylcyclohexyl)-ethyl]-4'-difluormethoxybiphenyl
4-[2-(trans-4-octylcyclohexyl)-ethyl]-4'-difluormethoxybiphenyl
4-[2-(trans-4-nonylcyclohexyl)-ethyl]-4'-difluormethoxybiphenyl
4-[2-(trans-4-decylcyclohexyl)-ethyl]-4'-difluormethoxybiphenyl
4-Difluormethoxy-[2-(trans-4-methylcyclohexyl)-ethyl]-benzol
4-Difluormethoxy-[2-(trans-4-ethylcyclohexyl)-ethyl]-benzol, Fp: -37 °C, Kp (extrapoliert): -60 °C,
Δn = 0,033, Viskosität: 6
4-Difluormethoxy-[2-(trans-4-propylcyclohexyl)-ethyl]-benzol, Fp: -2 °C, Kp: -14,5 °C, Δn = 0,042, Viskosität: 6
4-Difluormethoxy-[2-(trans-4-butylcyclohexyl)-ethyl]-benzol
4-Difluormethoxy-[2-(trans-4-pentylcyclohexyl)-ethyl]-benzol, Fp: 4 °C, Kp: 5,1 °C,Δn: 0,065, Viskosität: 7
4-Difluormethoxy-[2-(trans-4-hexylcyclohexyl)-ethyl]-benzol
4-Difluormethoxy-[2-(trans-4-heptylcyclohexyl)-ethyl)-benzol, Fp: 30 °C, Kp: 16,9 °C, Δn = 0,065, Viskosität: 8
4-Difluormethoxy-[2-(trans-4-octylcyclohexyl)-ethyl]-benzol
4-Difluormethoxy-[2-(trans-4-nonylcyclohexyl)-ethyl]-benzol
4-Difluormethoxy-[2-(trans-4-decylcyclohexyl)-ethyl]-benzol

### Beispiel 7

a) 4-(1H,1H-Difluorethoxy)-benzaldehyd
   Zu einer Suspension von 3,3 g Natriumhydrid in 100 ml 1,3-Dimethyl-2-imidazolidinon wird 16,4 g 2,2-Difluorethanol gegeben und das Gemisch 2 h bei 30 °C gerührt. Man kühlt auf 0-10 °C ab und gibt 12,4 g 4-Fluorbenzaldehyd zu. Es wird 2 h bei 5 °C und 4 h bei 90 °C gerührt. Nach Abkühlen wird das Reaktionsgemisch auf 200 ml 5%ige HCl gegossen und mit Ether extrahiert. Die organische Phase wird über Na₂SO₄ getrocknet, eingedampft und der Rückstand destilliert. Man erhält eine farblose Flüssigkeit.
b) 4-{2-[4-(1H,1H-Difluorethoxy)-phenyl]-ethenyl}-4'-pentyl-trans,trans-bicyclohexyl
   Eine Suspension von 53 g 4'-Pentyl-trans,trans-bicyclohexyl-4-methyl-triphenylphosphoniumiodid in 250 ml THF wird unter Eiskühlung mit 17,1 g des Produktes 7a und 9,3 g KOT versetzt. Die Mischung wird 1 h bei 5 °C gerührt. Danach wird 2 N HCl bis zur neutralen Reaktion der wäßrigen Phase und Wasser bis zur Lösung des Niederschlags zugegeben. Die organische Phase wird abgetrennt, über Na₂SO₄ getrocknet und eingedampft. Der Rückstand wird über eine Kieselgelsäule mit Petrolether/Ethylacetat 9:1 als Laufmittel chromatographiert. Man erhält farblose Kristalle.

Analog werden hergestellt:
4-{2-[4-(1H,1H-Difluorethoxy)-phenyl]-ethenyl}-4'-ethyl-trans,trans-bicyclohexyl
4-{2-[4-(1H,1H-Difluorethoxy)-phenyl]-ethenyl}-4'-propyl-trans,trans-bicyclohexyl
4-{2-[4-(1H,1H-Difluorethoxy)-phenyl]-ethenyl}-4'-butyl-trans,trans-bicyclohexyl
4-{2-[4-(1H,1H-Difluorethoxy)-phenyl]-ethenyl}-4'-hexyl-trans,trans-bicyclohexyl
4-{2-[4-(1H,1H-Difluorethoxy)-phenyl]-ethenyl}-4'-heptyl-trans,trans-bicyclohexyl
4-{2-[4-(1H,1H-Difluorethoxy)-phenyl]-ethenyl}-4'-octyl-trans,trans-bicyclohexyl
4-{2-[4-(1H,1H-Difluorethoxy)-phenyl]-ethenyl}-4'-nonyl-trans,trans-bicyclohexyl
4-{2-[4-(1H,1H-Difluorethoxy)-phenyl]-ethenyl}-4'-decyl-trans,trans-bicyclohexyl
4-Difluormethoxy-[2-(trans-4-methoxycyclohexyl)-ethenyl]-benzol
4-Difluormethoxy-[2-(trans-4-ethoxycyclohexyl)-ethenyl]-benzol
4-Difluormethoxy-[2-(trans-4-propoxycyclohexyl)-ethenyl]-benzol
4-Difluormethoxy-[2-(trans-4-butoxycyclohexyl)-ethenyl]-benzol
4-Difluormethoxy-[2-(trans-4-pentoxycyclohexyl)-ethenyl]-benzol
4-Difluormethoxy-[2-(trans-4-hexoxycyclohexyl)-ethenyl]-benzol
4-Difluormethoxy-[2-(trans-4-heptoxycyclohexyl)-ethenyl]-benzol
4-Difluormethoxy-[2-(trans-4-octoxycyclohexyl)-ethenyl]-benzol
4-Difluormethoxy-[2-(trans-4-nonoxycyclohexyl)-ethenyl]-benzol
4-Difluormethoxy-[2-(trans-4-decoxycyclohexyl)-ethenyl]-benzol

### Beispiel 8

### 4-{2-[4-1H,1H-Difluorethoxy)-phenyl]-ethyl}-4'-pentyl-trans,trans-bicyclohexyl

Eine Lösung von 16,8 g des Produkts 7b in 150 ml Essigester wird mit 10 g Pd/C (5 % Pd) versetzt und bei Raumtemperatur und Normaldruck hydriert. Der Katalysator wird abfiltriert, das Filtrat eingedampft und der Rückstand aus Ethanol umkristallisiert. Man erhält farblose Kristalle.

Analog werden hergestellt:
4-{2-[4-1H,1H-Difluorethoxy)-phenyl]ethyl}-4'-ethyl-trans,trans-bicyclohexyl
4-{2-[4-1H,1H-Difluorethoxy)-phenyl]-ethyl}-4'-propyl-trans,trans-bicyclohexyl
4-{2-[4-1H,1H-Difluorethoxy)-phenyl]-ethyl}-4'-butyl-trans,trans-bicyclohexyl
4-{2-[4-1H,1H-Difluorethoxy)-phenyl]-ethyl}-4'-hexyl-trans,trans-bicyclohexyl
4-{2-[4-1H,1H-Difluorethoxy)-phenyl]-ethyl}-4'-heptyl-trans,trans-bicyclohexyl
4-{2-[4-1H,1H-Difluorethoxy)-phenyl]-ethyl}-4'-octyl-trans,trans-bicyclohexyl
4-{2-[4-1H,1H-Difluorethoxy)-phenyl]-ethyl}-4'-nonyl-trans,trans-bicyclohexyl
4-{2-[4-1H,1H-Difluorethoxy)-phenyl]-ethyl}-4'-decyl-trans,trans-bicyclohexyl

### Beispiel 9

a) 3-[4-(trans-4-Propyl-cyclohexyl)-phenyl]-propansäure
   Eine Lösung von 12,6 g 3-[4-(trans-4-Propylcyclohexyl)-phenyl]-propensäure (erhalten durch Kondensation von 4-(trans-4-Propylcyclohexyl)-benzaldehyd mit Malonsäure in Pyridin) in 120 ml Ethylacetat wird mit 4 g Pd/C (5 % Pd) als Katalysator bei Raumtemperatur und Normaldruck hydriert. Der Katalysator wird abfiltriert und das Filtrat eingedampft. Man erhält farblose Kristalle.
b) 3-[4-(trans-4-Propyl-cyclohexyl)-phenyl]-propanol
   Zu einer auf 0 °C gehaltenen Suspension von 1.52 g Lithiumalanat in 200 ml THF wird eine Lösung von 11,3 g des Produkts 9a in 50 ml THF getropft. Nach 2 h Rühren bei Raumtemperatur wird das Reaktionsgemisch auf Wasser gegossen und mit 13 %iger HCl angesäuert. Das Gemisch wird mit Ether extrahiert, die organische Phase getrocknet und eingedampft. Man erhält farblose Kristalle.
c) 3-[4-(trans-4-Propyl-cyclohexyl)-phenyl]-propanal
   Zu einer auf -75 °C gehaltenen Lösung von 5,7 g Oxalylchlorid in 80 ml Dichlormethan tropft man eine Lösung von 7,2 g Dimethylsulfoxid in 12 ml Dichlormethan und rührt 5 min. Dann tropft man eine Lösung von 10,3 g des Produkts 9b in 20 ml Dichlormethan zu und rührt weitere 15 min. Dann gibt man 28 ml Triethylamin zu, läßt die Mischung auf 0 °C kommen und gibt 100 ml Wasser und 200 ml Ether zu. Die organische Phase wird abgetrennt, mit Wasser und gesättigter NaCl-Lösung gewaschen, über Na₂SO₄ getrocknet und eingedampft. Die Rückstand wird an einer Kieselgelsäule mit Petrolether/Ethylacetat 8:2 als Laufmittel chromatographiert. Man erhält ein farbloses Öl.
d) 1-(3,3-Difluorpropyl)-4-(trans-4-propylcyclohexyl)-benzol
   Zu einer Lösung von 7,9 g des Produktes 9c in 100 ml Hexan wird eine Lösung von 4,1 g DAST in 50 ml Hexan zugetropft und 2 h bei Raumtemperatur gerührt. Nach Zugabe von 100 ml Wasser wird die organische Phase abgetrennt, über Na₂SO₄ getrocknet und eingedampft. Der Rückstand wird an einer Kieselgelsäule mit Petrolether als Laufmittel chromatographiert und im Kugelrohrofen destilliert. Man erhält eine farblose Flüssigkeit. Fp: 12 °C, Kp: (-60 °C) extrapoliert, Δn = 0.032, Viskosität: 11.
   Analog werden hergestellt:
   1-(3,3-Difluorpropyl)-4-(trans-4-ethylcyclohexyl)-benzol
   1-(3,3-Difluorpropyl)-4-(trans-4-butylcyclohexyl)-benzol
   1-(3,3-Difluorpropyl)-4-(trans-4-pentylcyclohexyl)-benzol
   1-(3,3-Difluorpropyl)-4-(trans-4-hexylcyclohexyl)-benzol
   1-(3,3-Difluorpropyl)-4-(trans-4-heptylcyclohexyl)-benzol
   1-(3,3-Difluorpropyl)-4-(trans-4-octylcyclohexyl)-benzol
   1-(3,3-Difluorpropyl)-4-(trans-4-nonylcyclohexyl)-benzol
   1-(3,3-Difluorpropyl)-4-(trans-4-decylcyclohexyl)-benzol

### Beispiel 10

### 1H,1H-Difluorethyl-4'-pentyl-trans,trans-bicyclohexyl-4-carboxylat

Ein Gemisch aus 36,5 g 4'-Pentyl-trans,trans-bicyclohexyl-4-carbonsäure und 50 ml Thionylchlorid wird 1 h zum Sieden erhitzt. Überschüssiges Thionylchlorid wird abdestilliert, der Rückstand mit 50 ml Toluol versetzt und in einen Tropftrichter überführt. Diese Lösung wird zu einer Lösung aus 11,9 g 2,2-Difluorethanol und 43,3 ml Pyridin in 100 ml Toluol zugetropft. Das Reaktionsgemisch wird 1 h zum Sieden erhitzt und dannach 18 h bei Raumtemperatur belassen. Es werden 100 ml 2 N HCl zugefügt und die organische Phase abgetrennt. Die organische Phase wird zweimal mit Wasser gewaschen, über Na₂SO₄ getrocknet und eingedampft. Der Rückstand wird in 50 ml THF und 50 ml 5%igem wäßrigen Ammoniak aufgenommen und 1 h gerührt. Das Gemisch wird mit Petrolether extrahiert, über Na₂SO₄ getrocknet und eingedampft. Der Rückstand wird aus Acetonitril umkristallisiert. Man erhält farblose Kristalle.

Analog werden hergestellt:
1H,1H-Difluorethyl-4'-ethyl-trans,trans-bicyclohexyl-4-carboxylat
1H,1H-Difluorethyl-4'-propyl-trans,trans-bicyclohexyl-4-carboxylat
1H,1H-Difluorethyl-4'-butyl-trans,trans-bicyclohexyl-4-carboxylat
1H,1H-Difluorethyl-4'-hexyl-trans,trans-bicyclohexyl-4-carboxylat
1H,1H-Difluorethyl-4'-heptyl-trans,trans-bicyclohexyl-4-carboxylat
1H,1H-Difluorethyl-4'-octyl-trans,trans-bicyclohexyl-4-carboxylat
1H,1H-Difluorethyl-4'-nonyl-trans,trans-bicyclohexyl-4-carboxylat
1H,1H-Difluorethyl-4'-decyl-trans,trans-bicyclohexyl-4-carboxylat
1H,1H-Difluorethyl-trans-4-(4-methylphenyl)-cyclohexyl-carboxylat
1H,1H-Difluorethyl-trans-4-(4-ethylphenyl)-cyclohexyl-carboxylat
1H,1H-Difluorethyl-trans-4-(4-propylphenyl)-cyclohexyl-carboxylat
1H,1H-Difluorethyl-trans-4-(4-butylphenyl)-cyclohexyl-carboxylat
1H,1H-Difluorethyl-trans-4-(4-pentylphenyl)-cyclohexyl-carboxylat
1H,1H-Difluorethyl-trans-4-(4-hexylphenyl)-cyclohexyl-carboxylat
1H,1H-Difluorethyl-trans-4(4-heptylphenyl)-cyclohexyl-carboxylat
1H,1H-Difluorethyl-trans-4-(4-octylphenyl)-cyclohexyl-carboxylat
1H,1H-Difluorethyl-trans-4-(4-nonylphenyl)-cyclohexyl-carboxylat
1H,1H-Difluorethyl-trans-4-(4-decylphenyl)-cyclohexyl-carboxylat
1H,1H-Difluorethyl-trans-4-(4-methoxyphenyl)-cyclohexyl-carboxylat
1H,1H-Difluorethyl-trans-4-(4-ethoxyphenyl)-cyclohexyl-carboxylat
1H,1H-Difluorethyl-trans-4-(4-propoxyphenyl)-cyclohexyl-carboxylat
1H,1H-Difluorethyl-trans-4-(4-butoxyphenyl)-cyclohexyl-carboxylat
1H,1H-Difluorethyl-trans-4-(4-pentoxyphenyl)-cyclohexyl-carboxylat
1H,1H-Difluorethyl-trans-4-(3-fluor-4-pentoxyphenyl)-cyclohexyl-carboxylat
1H,1H-Difluorethyl-trans-4-(4-hexoxyphenyl)-cyclohexyl-carboxylat
1H,1H-Difluorethyl-trans-4-(4-heptoxyphenyl)-cyclohexyl-carboxylat
1H,1H-Difluorethyl-trans-4-(4-octoxyphenyl)-cyclohexyl-carboxylat
1H,1H-Difluorethyl-trans-4-(4-nonoxyphenyl)-cyclohexyl-carboxylat
1H,1H-Difluorethyl-trans-4-(4-decoxyphenyl)-cyclohexyl-carboxylat
1H,1H-Difluorethyl-4-(trans-4-ethylcyclohexyl)-benzoat
1H,1H-Difluorethyl-4-(trans-4-propylcyclohexyl)-benzoat
1H,1H-Difluorethyl-4-(trans-4-butylcyclohexyl)-benzoat
1H,1H-Difluorethyl-4-(trans-4-pentylcyclohexyl)-benzoat
1H,1H-Difluorethyl-4-(trans-4-hexylcyclohexyl)-benzoat
1H,1H-Difluorethyl-4-(trans-4-heptylcyclohexyl)-benzoat
1H,1H-Difluorethyl-4-(trans-4-octylcyclohexyl)-benzoat
1H,1H-Difluorethyl-4-(trans-4-nonylcyclohexyl)-benzoat
1H,1H-Difluorethyl-4-(trans-4-decylcyclohexyl)-benzoat

### Beispiel 11

a) 4-(tranS-4-Formyl-cyclohexyl)-4'-pentyl-biphenyl
   Zu einer Lösung von 33,2 g 4-(trans-4-Cyanocyclohexyl)-4'-pentyl-biphenyl in 1 l Pentan werden 112 ml einer 20%igen Lösungen von DIBALH in Hexan zugetropft und 18 h gerührt. Anschließend wird Wasser bis zum Nachlassen der Gasentwicklung, dann 200 ml 25%ige Schwefelsäure zugetropft. Die organische Phase wird abgetrennt, über Na₂SO₄ getrocknet und eingedampft. Man erhält farblose zerfließliche Kristalle.
b) 4-(trans-4-Difluormethyl-cyclohexyl)-4'-pentyl-biphenyl
   Zu einer Lösung von 18,0 g des Produkts 11a in 30 ml Dichlormethan wird bei Raumtemperatur eine Lösung von 6,9 g DAST in 10 ml Dichlormethan zugetropft und 18 h gerührt. Nach Zugabe von 150 ml Wasser wird die organische Phase abgetrennt, über Na₂SO₄ getrocknet und eingedampft. Der Rückstand wird an einer Kieselgelsäule mit Petrolether/Ethylacetat 95:5 chromatographiert. Man erhält farblose Kristalle.
   Analog werden hergestellt:
   4-(trans-4-Difluormethyl-cyclohexyl)-4'-ethyl-biphenyl
   4-(trans-4-Difluormethyl-cyclohexyl)-2',3'-difluor-4'-propyl-biphenyl
   4-(trans-4-Difluormethyl-cyclohexyl)-4'-propyl-biphenyl
   4-(trans-4-Difluormethyl-cyclohexyl)-4'-butyl-biphenyl
   4-(trans-4-Difluormethyl-cyclohexyl)-4'-hexyl-biphenyl
   4-(trans-4-Difluormethyl-cyclohexyl)-4'-heptyl-biphenyl
   4-(trans-4-Difluormethyl-cyclohexyl)-4'-octyl-biphenyl
   4-(trans-4-Difluormethyl-cyclohexyl)-4'-nonyl-biphenyl
   4-(trans-4-Difluormethyl-cyclohexyl)-4'-decyl-biphenyl
   4-(trans-4-Difluormethyl-cyclohexyl)-4'-methoxy-biphenyl
   4-(trans-4-Difluormethyl-cyclohexyl)-4'-ethoxy-biphenyl
   4-(trans-4-Difluormethyl-cyclohexyl)-3'-cyano-4'-ethoxy-biphenyl
   4-(trans-4-Difluormethyl-cyclohexyl)-4'-propoxy-biphenyl
   4-(trans-4-Difluormethyl-cyclohexyl)-4'-butoxy-biphenyl
   4-(trans-4-Difluormethyl-cyclohexyl)-4'-pentoxy-biphenyl
   4-(trans-4-Difluormethyl-cyclohexyl)-4'-hexoxy-biphenyl
   4-(trans-4-Difluormethyl-cyclohexyl)-4'-heptoxy-biphenyl
   4-(trans-4-Difluormethyl-cyclohexyl)-4'-octoxy-biphenyl
   4-(trans-4-Difluormethyl-cyclohexyl)-4'-nonoxy-biphenyl
   4-(trans-4-Difluormethyl-cyclohexyl)-4'-decoxy-biphenyl

### Beispiel 12

a) 4'-Propyl-trans,trans-bicyclohexyl-4-carbaldehyd
   Zu einer Suspension von 82,0 g PCC in 400 ml Dichlormethan wird eine Lösung von 59,6 g 4-Hydroxymethyl-4'-propyl-trans,trans-bicyclohexyl in 200 ml Dichlormethan zugetropft. Nach 2 h Rühren werden 200 ml Ether zugegeben. Das Gemisch wird filtriert und der Rückstand mit Ether gewaschen. Das Filtrat wird eingedampft und der Rückstand über eine kurze Kieselgelsäule mit Toluol als Laufmittel chromatographiert. Man erhält ein farbloses Öl.
b) 4-Difluormethyl-4'-propyl-trans,trans-bicyclohexyl
   Zu einer Lösung von 4,9 g des Produkts 12a in 100 ml Hexan wird bei Raumtemperatur eine Lösung von 2,7 ml DAST in 30 ml Hexan zugetropft und 18 h gerührt. Nach Zugabe von 100 ml Wasser wird die organische Phase abgetrennt, über Na₂SO₄ getrocknet und eingedampft. Der Rückstand wird an einer Kieselgelsäule mit Petrolether als Laufmittel chromatographiert:
   Man erhält eine farblose Flüssigkeit. Fp: 19 °C, Kp: 17 °C, Δn = 0.003, Viskosität: 10.
   Analog werden hergestellt:
   4-Difluormethyl-4'-ethyl-trans,trans-bicyclohexyl
   4-Difluormethyl-4'-butyl-trans,trans-bicyclohexyl
   4-Difluormethyl-4'-pentyl-trans,trans-bicyclohexyl Fp: 29 °C, Kp: 32.1 °C, Δn = 0.0, Viskosität: 14
   4-Difluormethyl-4'-hexyl-trans,trans-bicyclohexyl
   4-Difluormethyl-4'-heptyl-trans,trans-bicyclohexyl
   4-Difluormethyl-4'-octyl-trans,trans-bicyclohexyl
   4-Difluormethyl-4'-nonyl-trans,trans-bicyclohexyl
   4-Difluormethyl-4'-decyl-trans,trans-bicyclohexyl

### Beispiel 13

a) 1-Difluormethoxy-4-jod-benzol
   In eine Mischung aus 300 ml THF und 30 ml Wasser werden 100 g (0,455 mol) 4-Jodphenol und 54,6 g (1,365 mol) Natriumhydroxid gegeben. Nach ca. 1/2 h Rühren wird die Mischung im Rotationsverdampfer eingedampft; dies wird nach Zugabe von 200 ml Toluol wiederholt. Der Rückstand wird in 400 ml THF aufgenommen. Unter Kühlung auf 0 °C werden 47,1 g (0,545 mol) Chlordifluormethan eingeleitet und dann 1 h gerührt. Danach wurde 18 h bei ca. +5 °C gerührt. Von dem entstandenen schleimigen Niederschlag wurde die überstehende Lösung abdekantiert. Sie wurde im Rotationsverdampfer eingedampft, der Rückstand über eine Kieselgel-Säule mit Petrolether als Laufmittel chromatographiert.
   Analog werden hergestellt:
   1-Difluormethoxy-4-brom-benzol
   1-Difluormethoxy-2-fluor-4-brom-benzol
b) 4-Difluormethoxy-4'-(trans-4-pentylcyclohexyl)-tolan
   Eine Lösung von 15,3 g 4-(trans-4-pentylcyclohexyl)-phenylacetylen (erhältlich aus dem Acetophenon durch Dehydratisierung mit PCl₅/KOT), 16,2 g Difluormethoxy-4-iod-benzol, 100 ml Triethylamin, 0,085 g Bis-(triphenylphosphin)palladiumdichlorid und 0,11 g Kupfer(I)iodid wird 3 Stunden bei Raumtemperatur gerührt. Das trübe Gemisch wird in verd. Salzsäure (500 ml Wasser + 200 ml 37%ige Salzsäure) eingerührt, mit tertiär Butylmethylether extrahiert und wie üblich aufgearbeitet. Fp: 62 °C, Kp: 203,1 °C
   Analog werden hergestellt:
   4-Difluormethoxy-4'-(trans-4-methylcyclohexyl)-tolan
   4-Difluormethoxy-4'-(trans-4-ethylcyclohexyl)-tolan
   4-Difluormethoxy-4'-(trans-4-propylcyclohexyl)-tolan,
   Fp: 87 °C, Kp: 212 °C
   4-Difluormethoxy-4'-(trans-4-butylcyclohexyl)-tolan
   4-Difluormethoxy-4'-(trans-4-pentylcyclohexyl)-tolan
   4-Difluormethoxy-4'-(trans-4-hexylcyclohexyl)-tolan
   4-Difluormethoxy-4'-(trans-4-heptylcyclohexyl)-tolan
   4-Difluormethoxy-4'-(trans-4-octylcyclohexyl)-tolan
   4-Difluormethoxy-4'-(trans-4-nonylcyclohexyl)-tolan
   4-Difluormethoxy-4'-(trans-4-decylcyclohexyl)-tolan
   4-Difluormethoxy-4'-[2-(trans-4-methylcyclohexyl)-ethyl]-tolan
   4-Difluormethoxy-4'-[2-(trans-4-ethylcyclohexyl)-ethyl]-tolan
   4-Difluormethoxy-4'-[2-(trans-4-propylcyclohexyl)-ethyl)-tolan
   4-Difluormethoxy-4'-[2-(trans-4-butylcyclohexyl)-ethyl]-tolan
   4-Difluormethoxy-4'-[2-(trans-4-pentylcyclohexyl)-ethyl]-tolan
   4-Difluormethoxy-4'-[2-(trans-4-hexylcyclohexyl)-ethyl]-tolan
   4-Difluormethoxy-4'-[2-(trans-4-heptylcyclohexyl)-ethyl]-tolan
   4-Difluormethoxy-4'-[2-(trans-4-octylcyclohexyl)-ethyl]-tolan
   4-Difluormethoxy-4'-[2-(trans-4-nonylcyclohexyl)-ethyl]-tolan
   4-Difluormethoxy-4'-[2-(trans-4-decylcyclohexyl)-ethyl]-tolan
   4-Difluormethoxy-4'-methyl-tolan
   4-Difluormethoxy-4'-ethyl-tolan
   4-Difluormethoxy-4'-propyl-tolan,
   Fp: 50 °C, Kp (extrapoliert): 10 °C, Viskotität: 7
   4-Difluormethoxy-4'-butyl-tolan
   4-Difluormethoxy-4'-pentyl-tolan
   4-Difluormethoxy-4'-hexyl-tolan
   4-Difluormethoxy-4'-heptyl-tolan
   4-Difluormethoxy-4'-octyl-tolan
   4-Difluormethoxy-4'-nonyl-tolan
   4-Difluormethoxy-4'-decyl-tolan
   Analog werden hergestellt mit 4-(Difluormethylthio)-iodbenzol (erhältlich durch Chlorsulfonylierung von Iodbenzol, Reduktion zum 4-Thiolo-iodbenzol mit Zink in verdünnter Salzsäure und Veretherung analog Beispiel 1):
   4-Difluormethylthio-4'-(trans-4-methylcyclohexyl)-tolan
   4-Difluormethylthio-4'-(trans-4-ethylcyclohexyl)-tolan
   4-Difluormethylthio-4'-(trans-4-propylcyclohexyl)-tolan
   4-Difluormethylthio-4'-(trans-4-butylcyclohexyl)-tolan
   4-Difluormethylthio-4'-(trans-4-pentylcyclohexyl)-tolan
   4-Difluormethylthio-4'-(trans-4-hexylcyclohexyl)-tolan
   4-Difluormethylthio-4'-(trans-4-heptylcyclohexyl)-tolan
   4-Difluormethylthio-4'-(trans-4-octylcyclohexyl)-tolan
   4-Difluormethylthio-4'-(trans-4-nonylcyclohexyl)-tolan
   4-Difluormethylthio-4'-(trans-4-decylcyclohexyl)-tolan

### Beispiel 14

### 4-(2,2-Difluorethyl)-4'-pentyl-trans,trans-bicyclohexyl

13 g 4-(2-Oxoethyl)-4'-pentyl-trans,trans-bicyclohexyl (erhältlich aus der Bicyclohexylcarbonsäure, Reduktion zum Alkohol, Umwandlung in das Nitril und Überführung mit DIBALH in den Aldehyd) wird in 100 ml Hexan gelöst. Bei Raumtemperatur wird eine Lösung von 6,2 ml DAST in 100 ml Hexan zugetropft und 10 Stunden bei Raumtemperatur weitergerührt. Anschließend wird wie üblich aufgearbeitet. Fp: -1 °C, Kp: 82 °C, Δn = 0,040, Viskosität: 11
Analog werden hergestellt:
4-(2,2-Difluorethyl)-4'-methyl-trans,trans-bicyclohexyl
4-(2,2-Difluorethyl)-4'-ethyl-trans,trans-bicyclohexyl
4-(2,2-Difluorethyl)-4'-propyl-trans,trans-bicyclohexyl
4-(2,2-Difluorethyl)-4'-butyl-trans,trans-bicyclohexyl
4-(2,2-Difluorethyl)-4'-hexyl-trans,trans-bicyclohexyl
4-(2,2-Difluorethyl)-4'-heptyl-trans,trans-bicyclohexyl
4-(2,2-Difluorethyl)-4'-octyl-trans,trans-bicyclohexyl
4-(2,2-Difluorethyl)-4'-nonyl-trans,trans-bicyclohexyl
4-(2,2-Difluorethyl)-4'-decyl-trans,trans-bicyclohexyl

### Beispiel 15

### 1-(4-Ethoxyphenyl)-2-(4'-difluormethoxyphenyl)-ethen

22,2 g 4-Ethoxystyrol, 33,5 g 4-(Difluormethoxy)-brombenzol (erhältlich aus 4-Bromphenol analog Beispiel 1)), 0,67 g Palladium(II)acetat, 20,8 ml Triethylamin und 1,83 g Tri-o-tolylphosphin werden in 225 ml Acetonitril 24 Stunden zum Sieden erhitzt. Nach dem Abkühlen auf 0 °C werden die Kristalle abgesaugt und mit Acetonitril und Wasser gewaschen. Fp: 177 °C
Analog werden hergestellt:
1-(4-Methoxyphenyl)-2-(4'-difluormethoxyphenyl)-ethen
1-(4-Propoxyphenyl)-2-(4'-difluormethoxyphenyl)-ethen
1-(4-Butoxyphenyl)-2-(4'-difluormethoxyphenyl)-ethen
1-(4-Pentoxyphenyl)-2-(4'-difluormethoxyphenyl)-ethen
1-(4-Hexoxyphenyl)-2-(4'-difluormethoxyphenyl)-ethen
1-(4-Heptoxyphenyl)-2-(4'-difluormethoxyphenyl)-ethen
1-(4-Octoxyphenyl)-2-(4'-difluormethoxyphenyl)-ethen
1-(4-Nonoxyphenyl)-2-(4'-difluormethoxyphenyl)-ethen
1-(4-Decoxyphenyl)-2-(4'-difluormethoxyphenyl)-ethen
1-[4-(trans-4-Methylcyclohexyl)-phenyl]-2-(4'difluormethoxyphenyl)-ethen
1-[4-(trans-4-Ethylcyclohexyl)-phenyl]-2-(4'difluormethoxyphenyl)-ethen
1-[4-(trans-4-Propylcyclohexyl)-phenyl]-2-(4'difluormethoxyphenyl)-ethen
1-[4-(trans-4-Butylcyclohexyl)-phenyl]-2-(4'difluormethoxyphenyl)-ethen
1-[4-(trans-4-Pentylcyclohexyl)-phenyl]-2-(4'difluormethoxyphenyl)-ethen
1-[4-(trans-4-Hexylcyclohexyl)-phenyl]-2-(4'difluormethoxyphenyl)-ethen
1-[4-(trans-4-Heptylcyclohexyl)-phenyl]-2-(4'difluormethoxyphenyl)-ethen
1-[4-(trans-4-Octylcyclohexyl)-phenyl]-2-(4'difluormethoxyphenyl)-ethen
1-[4-(trans-4-Nonylcyclohexyl)-phenyl]-2-(4'difluormethoxyphenyl)-ethen
1-[4-(trans-4-Decylcyclohexyl)-phenyl]-2-(4'difluormethoxyphenyl)-ethen
1-[4-(5-Methyl-1,3-pyrimidin-2-yl)-phenyl]-2-(4'-difluormethoxyphenyl)-ethen
1-[4-(5-Ethyl-1,3-pyrimidin-2-yl)-phenyl]-2-(4'-difluormethoxyphenyl)-ethen
1-[4-(5-Propyl-1,3-pyrimidin-2-yl)-phenyl]-2-(4'-difluormethoxyphenyl)-ethen
1-[4(5-Butyl-1,3-pyrimidin-2-yl)-phenyl]-2-(4,-difluormethoxyphenyl)-ethen
1-[4-(5-Pentyl-1,3-pyrimidin-2-yl)-phenyl]-2-(4'-difluormethoxyphenyl)-ethen
1-[4-(5-Hexyl-1,3-pyrimidin-2-yl)-phenyl]-2(4'-difluormethoxyphenyl)-ethen
1-[4-(5-Heptyl-1,3-pyrimidin-2-yl)-phenyl]-2-(4'-difluormethoxyphenyl)-ethen
1-[4-(5-Octyl-1,3-pyrimidin-2-yl)-phenyl]-2-(4'-difluormethoxyphenyl)-ethen
1-[4-(5-Nonyl-1,3-pyrimidin-2-yl)-phenyl]-2-(4'-difluormethoxyphenyl)-ethen
1-[4-(5-Decyl-1,3-pyrimidin-2-yl)-phenyl]-2-(4'-difluormethoxyphenyl)-ethen

### Beispiel 16

### 1-(4-Ethoxyphenyl)-2-(4'difluormethoxyphenyl)-ethan

19,7 g 1-(4-Ethoxyphenyl)-2-(4'difluormethoxyphenyl)-ethen werden in 150 ml THF mit 6 g Pd/C (5 % Pd) als Katalysator analog Beispiel 6b hydriert. Fp: 37 °C, Δn = 0,103, Viskosität: 8
Analog werden hergestellt:
1-(4-Methoxyphenyl)-2-(4'-difluormethoxphenyl)-ethan
1-(4-Propoxyphenyl)-2-(4'-difluormethoxphenyl)-ethan
1-(4-Butoxyphenyl)-2-(4'-difluormethoxyphenyl)-ethan.
1-(4-Pentoxphenyl)-2-(4'-difluormethoxphenyl)-ethan
1-(4-Hexoxyphenyl)-2-(4'-difluormethoxyphenyl)-ethan
1-(4-Heptoxyphenyl)-2-(4'-difluormethoxyphenyl)-ethan
1-(4-Octoxyphenyl)-2-(4'-difluormethoxyphenyl)-ethan
1-(4-Nonoxyphenyl)-2-(4'-difluormethoxyphenyl)-ethan
1-(4-Decoxyphenyl)-2-(4'-difluormethoxyphenyl)-ethan
1-(4-Methylphenyl)-2-(4-difluormethoxybiphenyl-4'-yl)-ethan
1-(4-Ethylphenyl)-2-(4-difluormethoxybiphenyl-4'-yl)-ethan
1-(4-Propylphenyl)-2-(4-difluormethoxybiphenyl-4'-yl)-ethan
1-(4-Butylphenyl)-2-(4-difluormethoxybiphenyl-4'-yl)-ethan
1-(4-Pentylphenyl)-2-(4-difluormethoxybiphenyl-4'-yl)-ethan
1-(4-Hexylphenyl)-2-(4-difluormethoxybiphenyl-4'-yl)-ethan
1-(4-Heptylphenyl)-2-(4-difluormethoxybiphenyl-4'-yl)-ethan
1-(4-Octylphenyl)-2-(4-difluormethoxybiphenyl-4'-yl)-ethan
1-(4-Nonylphenyl)-2-(4-difluormethoxybiphenyl-4'-yl)-ethan
1-(4-Decylphenyl)-2-(4-difluormethoxybiphenyl-4'-yl)-ethan
1-(4-Methoxyphenyl)-2-(4-difluormethoxybiphenyl-4'-yl)-ethan
1-(4-Ethoxyphenyl)-2-(4-difluormethoxybiphenyl-4'-yl)-ethan, Fp: 153°, Kp (extrapoliert): 110°, Δn: 0,118
1-(4-Propoxyphenyl)-2-(4-difluormethoxybiphenyl-4'-yl)-ethan
1-(4-Butoxyphenyl)-2-(4-difluormethoxybiphenyl-4'-yl)-ethan
1-(4-Pentoxyphenyl)-2-(4-difluormethoxybiphenyl-4'-yl)-ethan
1-(4-Hexoxyphenyl)-2-(4-difluormethoxybiphenyl-4'-yl)-ethan
1-(4-Heptoxyphenyl)-2-(4-difluormethoxybiphenyl-4'-yl)-ethan
1-(4-Octoxyphenyl)-2-(4-difluormethoxybiphenyl-4'-yl)-ethan
1-(4-Nonoxyphenyl)-2-(4-difluormethoxybiphenyl-4'-yl)-ethan
1-(4-Decoxyphenyl)-2-(4-difluormethoxybiphenyl-4'-yl)-ethan
1-[4-(trans-4-Methylcyclohexyl)-phenyl]-2-(4'-difluormethoxyphenyl)-ethan
1-[4-(trans-4-Ethylcyclohexyl)-phenyl]-2-(4'-difluormethoxyphenyl)-ethan
1-[4-(trans-4-Propylcyclohexyl)-phenyl]-2-(4'-difluormethoxyphenyl)-ethan, Fp: 51 °C, Kp: 79,6 °C, Δn = 0,125
1-[4-(trans-4-Butylcyclohexyl)-phenyl]-2-(4'-difluormethoxyphenyl)-ethan
1-[4-(trans-4-Pentylcyclohexyl)-phenyl]-2-(4'-difluormethoxyphenyl)-ethan, Fp: 32 °C, Kp: 90,2 °C, Δn = 0,123
1-[4-(trans-4-Hexylcyclohexyl)-phenyl]-2-(4'-difluormethoxyphenyl)-ethan
1-[4-(trans-4-Heptylcyclohexyl)-phenyl]-2-(4'-difluormethoxyphenyl)-ethan
1-[4-(trans-4-Octylcyclohexyl)-phenyl]-2-(4'-difluormethoxyphenyl)-ethan
1-[4-(trans-4-Nonylcyclohexyl)-phenyl]-2-(4'-difluormethoxyphenyl)-ethan
1-[4-(trans-4-Decylcyclohexyl)-phenyl]-2-(4'-difluormethoxyphenyl)-ethan
1-[4-(trans-4-Methylcyclohexyl)-phenyl]-2-(4'-difluormethylthiophenyl)-ethan
1-[4-(trans-4-Ethylcyclohexyl)-phenyl]-2-(4'-difluormethylthiophenyl)-ethan
1-[4-(trans-4-Propylcyclohexyl)-phenyl]-2-(4'-difluormethylthiophenyl)-ethan
1-[4-(trans-4-Butylcyclohexyl)-phenyl]-2-(4'-difluormethylthiophenyl)-ethan
1-[4-(trans-4-Pentylcyclohexyl)-phenyl]-2-(4'-difluormethylthiophenyl)-ethan
1-[4-(trans-4-Hexylcyclohexyl)-phenyl]-2-(4'-difluormethylthiophenyl)-ethan
1-[4-(trans-4-Heptylcyclohexyl)-phenyl]-2-(4'-difluormethylthiophenyl)-ethan
1-[4-(trans-4-Octylcyclohexyl)-phenyl]-2-(4'-difluormethylthiophenyl)-ethan
1-[4-(trans-4-Nonylcyclohexyl)-phenyl]-2-(4'-difluormethylthiophenyl)-ethan
1-[4-(trans-4-Decylcyclohexyl)-phenyl]-2-(4'-difluormethylthiophenyl)-ethan
1-[4-(5-Methyl-1,3-pyrimidin-2-yl)-phenyl]-2-(4'-difluormethoxyphenyl)-ethan
1-[4-(5-Ethyl-1,3-pyrimidin-2-yl)-phenyl]-2-(4'-difluormethoxyphenyl)-ethan
1-[4-(5-Propyl-1,3-pyrimidin-2-yl)-phenyl]-2-(4'-difluormethoxyphenyl)-ethan
1-[4-(5-Butyl-1,3-pyrimidin-2-yl)-phenyl]-2-(4'-difluormethoxyphenyl)-ethan
1-[4-(5-Pentyl-1,3-pyrimidin-2-yl)-phenyl]-2-(4'-difluormethoxyphenyl)-ethan
1-[4-(5-Hexyl-1,3-pyrimidin-2-yl)-phenyl]-2-(4'-difluormethoxyphenyl)-ethan
1-[4-(5-Heptyl-1,3-pyrimidin-2-yl)-phenyl]-2-(4'-difluormethoxyphenyl)-ethan
1-[4-(5-Octyl-1,3-pyrimidin-2-yl)-phenyl]-2-(4'-difluormethoxyphenyl)-ethan
1-[4-(5-Nonyl-1,3-pyrimidin-2-yl)-phenyl]-2-(4'-difluormethoxyphenyl)-ethan
1-[4-(5-Decyl-1,3-pyrimidin-2-yl)-phenyl]-2-(4'-difluormethoxyphenyl)-ethan

### Beispiel 17

### 4-(trans-4-Propylcyclohexyl)-phenyl-4-difluormethoxybenzoat

4-Difluormethoxybenzoesäure (erhältlich durch Veretherung der phenolischen OH-Gruppe von p-Hydroxybenzylalkohol analog Beispiel 1 und anschließender Oxidation mit Kaliumpermanganat) wird, wie in Beispiel 5 b) angegeben, mit 4-(trans-4-Propylcyclohexyl)-phenol nach der DCC-Methode verestert. Fp: 104 °C, Kp: 193,5 °C.

Analog werden hergestellt:
4-(trans-4-Methylcyclohexyl)-phenyl-4-difluormethoxybenzoat
4-(trans-4-Ethylcyclohexyl)-phenyl-4-difluormethoxybenzoat
4-(trans-4-Butylcyclohexyl)-phenyl-4-difluormethoxybenzoat
4-(trans-4-Pentylcyclohexyl)-phenyl-4-difluormethoxybenzoat
4-(trans-4-Hexylcyclohexyl)-phenyl-4-difluormethoxybenzoat
4-(trans-4-Heptylcyclohexyl)-phenyl-4-difluormethoxybenzoat
4-(trans-4-Nonylcyclohexyl)-phenyl-4-difluormethoxybenzoat
4-(trans-4-Octylcyclohexyl)-phenyl-4-difluormethoxybenzoat
4-(trans-4-Decylcyclohexyl)-phenyl-4-difluormethoxybenzoat
4-Methylphenyl-4-difluormethoxybenzoat
4-Eethylphenyl-4-difluormethoxybenzoat
4-Propylphenyl-4-difluormethoxybenzoat
4-Butylphenyl-4-difluormethoxybenzoat
4-Pentylphenyl-4-difluormethoxybenzoat
4-Hexylphenyl-4-difluormethoxybenzoat
4-Heptylphenyl-4-difluormethoxybenzoat
4-Octylphenyl-4-difluormethoxybenzoat
4-Nonylphenyl-4-difluormethoxybenzoat
4-Decylphenyl-4-difluormethoxybenzoat
4'-Methylbiphenyl-4-yl-4-difluormethoxybenzoat
4'-Ethylbiphenyl-4-yl-4-difluormethoxybenzoat
4'-Propylbiphenyl-4-yl-4-difluormethoxybenzoat
4'-Butylbiphenyl-4-yl-4-difluormethoxybenzoat
4'-Pentylbiphenyl-4-yl-4-difluormethoxybenzoat
4'-Hexylbiphenyl-4-yl-4-difluormethoxybenzoat
4'-Heptylbiphenyl-4-yl-4-difluormethoxybenzoat
4'-Octylbiphenyl-4-yl-4-difluormethoxybenzoat
4'-Nonylbiphenyl-4-yl-4-difluormethoxybenzoat
4'-Decylbiphenyl-4-yl-4-difluormethoxybenzoat
Mit 4-Difluormethylthiobenzoesäure (erhältlich aus p-Toluolsulfonsäure durch Oxidation zur Carbonsäure, Veresterung, Überführung in das Sulfonylchlorid, Reduktion mit Zink/Salzsäure, Veretherung analog Beispiel 1 und Freisetzen der Säure) erhält man analog:
4-(trans-4-Methylcyclohexyl)-phenyl-4-difluormethylthiobenzoat
4-(trans-4-Ethylcyclohexyl)-phenyl-4-difluormethylthiobenzoat
4-(trans-4-Propylcyclohexyl)-phenyl-4-difluormethylthiobenzoat
4-(trans-4-Butylcyclohexyl)-phenyl-4-difluormethylthiobenzoat
4-(trans-4-Pentylcyclohexyl)-phenyl-4-difluormethylthiobenzoat
4-(trans-4-Hexylcyclohexyl)-phenyl-4-difluormethylthiobenzoat
4-(trans-4-Heptylcyclohexyl)-phenyl-4-difluormethylthiobenzoat
4-(trans-4-Octylcyclohexyl)-phenyl-4-difluormethylthiobenzoat
4-(trans-4-Nonylcyclohexyl)-phenyl-4-difluormethylthiobenzoat
4-(trans-4-Decylcyclohexyl)-phenyl-4-difluormethylthiobenzoat
Folgende Beispiele betreffen erfindungsgemäße flüssigkristalline Phasen.

### Beispiel A:

Eine flüssigkristalline Phase, bestehend aus:
10 % p-trans-4-Propylcyclohexyl-benzonitril
10 % p-trans-4-Butylcyclohexyl-benzonitril
10 % p-trans-4-Pentylyclohexyl-benzonitril
20 % Difluormethoxy-4-(trans-4-pentylcyclohexyl)-benzol
10 % trans-1-p-Ethoxyphenyl-4-propylcyclohexan
8 % trans-1-p-Butoxyphenyl-4-propylcyclohexan
5 % trans-4-(trans-4-propylcyclohexyl)-cyclohexancarbonsäure-4'-p-propylphenyl-ester
5 % trans-4-(trans-4-Propylcyclohexyl)-cyclohexancarbonsäure-4'-p-pentylphenyl-ester
5 % trans-4-(trans-4-Butylcyclohexyl)-cyclohexancarbonsäure-4'-p-propylphenyl-ester
5 % trans-4-(trans-4-Butylcyclohexyl)-cyclohexancarbonsäure-4'-p-pentylphenyl-ester
4 % 4-(trans-4-Propylcyclohexyl)-2'-fluor-4'-(trans-4-propylcyclohexyl)-biphenyl
4 % 2-Flour-4-(trans-4-pentylcyclohexyl)-4'-(trans-4-propylcyclohexyl)-biphenyl
und
4 % 4-(trans-4-Pentylcyclohexyl)-2'-fluor-4'-(trans-4-pentylcyclohexyl)-biphenyl
hat einen Schmelzpunkt von -18.8 °C, einen Klärpunkt von 78 °C und eine Viskosität von 16 mm²/s bei 20 °C.

### Beispiel B:

Eine flüssigkristalline Phase, bestehend aus
15 % Difluormethoxy-4-(trans-4-propylcyclohexyl)-benzol
17 % Difluormethoxy-4-(trans-4-pentylcyclohexyl)-benzol
13 % Difluormethoxy-4-(trans-4-heptylcyclohexyl)-benzol
10 % trans-1-p-Methoxyphenyl-4-propylcyclohexan
10 % trans-1-p-Ethoxyphenyl-4-propylcyclohexan
5 % trans-1-p-Butoxyphenyl-4-propylcyclohexan
5 % 4-(trans-4-Propylcyclohexyl)-2'-fluor-4'-(trans-4-propylcyclohexyl)-biphenyl
5 % 2-Fluor-4-(trans-4-pentylcylcohexyl)-4'-(trans-4-propylcyclohexyl)-biphenyl
5 % 4-(trans-4-Pentylcyclohexyl)-2'-fluor-4'-(trans-4-pentylcyclohexyl)-biphenyl
5 % 4,4'-Bis-(trans-4-propylcyclohexyl)-biphenyl
5 % 4-(trans-4-Pentylcyclohexyl)-4'-(trans-4-propylcyclohexyl)-biphenyl
und
5 % 4,4'-Bis-(trans-4-pentylcyclohexyl)-biphenyl
hat einen Schmelzpunkt von -20.8 °C, einen Klärpunkt von 72 °C und eine Viskosität von 12 mm²/s bei 20 °C

### Beispiel C:

Eine flüssigkristalline Phase, bestehend aus
16 % p-trans-4-Propylcyclohexyl-benzonitril
4 % p-trans-4-Pentylcyclohexyl-benzonitril
18 % 1-(3,3-Difluorpropyl)-4-(trans-4-propylcyclohexyl)-benzol
10 % trans-1-p-Methoxyphenyl-4-propylcyclohexan
10 % trans-1-p-Ethoxyphenyl-4-propylcyclohexan
11 % 4-Ethyl-4'-(trans-4-propylcyclohexyl)-biphenyl
11 % 4-Ethyl-4'-(trans-4-pentylcyclohexyl)-biphenyl
3 % 4-(trans-4-Propycyclohexyl)-2'-fluor-4'-(trans-4-propylcyclohexyl)-biphenyl
4 % 2-Fluor-4-(trans-4-pentylcyclohexyl)-4'-(trans-4-propylcyclohexyl)-biphenyl
3 % 4-(trans-4-Pentylcyclohexyl)-2'-fluor-4'-(trans-4-pentylcyclohexyl)-biphenyl
3 % 4,4'-Bis-(trans-4-propylcyclohexyl)-biphenyl
4 % 4-(trans-4-Pentylcyclohexyl)-4'-(trans-4-propylcyclohexyl)-biphenyl und
3 % 4,4'-Bis-(trans-4-pentylcyclohexyl)-biphenyl
hat einen Schmelzpunkt von -10.3 °C, einen Klärpunkt von 91 °C und einen Viskosität von 17 mm²/s bei 20 °C.

### Beispiel D:

Eine flüssigkristalline Phase bestehend aus
24 % p-trans-4-Propylcyclohexyl-benzonitril
36 % p-trans-4-Pentylcyclohexyl-benzonitril
25 % p-trans-4-Heptylcyclohexyl-bezonitril
15 % 4-Cyan-4'-(trans-4-pentylcyclohexyl)-biphenyl
hat einen Klärpunkt von 71 °C, ein Δn von 0,140 und eine Viskosität von 28.

Diese Phase wird mit 10 % einer der folgenden erfindungsgemäßen Verbindungen I gemischt. Die erhaltenen Werte dieser erfindungsgemäßen Phase sind in untenstehender Tabelle 1 zusammengefaßt.

### Verbindungen I

- 1: 4-Difluormethoxy-4'-octoxy-biphenyl
- 2: Difluormethoxy-4-(trans-4-propylcyclohexyl)-benzol
- 3: Difluormethoxy-4-(trans-4-pentylcyclohexyl)-benzol
- 4: 4-[2-(4-Difluormethoxyphenyl)-ethyl]-4'-pentyl-trans,trans-bicyclohexyl
- 5: Difluormethoxy-4-(5-heptyl-1,3-pyrimidin-2-yl)-benzol
- 6: 4-Difluormethoxy-4'-(trans-4-pentylcyclohexyl)-biphenyl

**Tabelle 1**

| I | Kp [°C] | Δn | Viskosität |
|---|---|---|---|
| 1 | 65,4 | 0,1349 | 26,8 |
| 2 | 60,2 | 0,1291 | 23,8 |
| 3 | 61,8 | 0,1313 | 24,5 |
| 4 | 76,7 | 0,1353 | 27,4 |
| 5 | 63,7 | 0,1368 | 26,7 |
| 6 | 76,1 | 0,1411 | 28,2 |

### Beispiel E:

Eine flüssigkristalline Phase bestehend aus
22 % trans-1-p-Ethylphenyl-4-propylcyclohexan
20 % trans-1-p-Methoxyphenyl-4-propylcyclohexan
15 % trans-1-p-Ethoxyphenyl-4-propylcyclohexan
19 % 4-Ethyl-4'-(trans-4-propylcyclohexyl)-biphenyl
14 % 4-Ethyl-4'-(trans-4-pentylcyclohexyl)-biphenyl
5 % 4-(trans-4-Propylcyclohexyl)-4'-(trans-4-propylcyclohexyl)-biphenyl
5 % 4-(trans-4-Pentylcyclohexyl)-4'-(trans-4-propylcyclohexyl)-biphenyl
hat einen Klärpunkt von 72 °C, ein Δn von 0,1131 und eine Viskosität von 11.

Diese Phase wird mit 10 % einer der folgenden erfindungsgemäßen Verbindungen I gemischt. Die erhaltenen Werte dieser erfindungsgemäßen Phase sind in untenstehender Tabelle 2 zusammengefaßt.

### Verbindungen I

- 7: 4-Difluormethoxy-phenyl-trans-4-pentyl-cyclohexyl-carboxylat
- 8: 4-Difluormethoxy-phenyl-4'-pentyl-trans,trans-bicyclohexyl-4-carboxylat

**Tabelle 2**

| I | Kp [°C] | Δn | Viskosität |
|---|---|---|---|
| 7 | 67,8 | 0,1080 | 11,9 |
| 8 | 82,7 | 0,1108 | 12,6 |

## Patentansprüche

1. Difluormethylverbindungen der Formel I,
R-(A¹-Z¹-)ₘ-A²Q-CHF₂ I
worin
R Halogen, -CN, -NCS, einen geradkettigen Alkyl- oder Alkenylrest mit 1 bis 15 C-Atomen, wobei in diesen Resten auch eine oder mehrere CH₂-Gruppen jeweils unabhängig voneinander durch -O-, -S-, 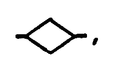 -CO-, -CO-O-, -O-CO- oder -O-CO-O- so ersetzt sein können, daß O-Atome nicht direkt miteinander verknüpft sind, oder einen geradkettigen Perfluoralkylrest, in dem auch Fluor partiell durch H ersetzt sein kann,
A¹ und A² jeweils unabhängig voneinander einen
(a) trans-1,4-Cyclohexylenrest, worin auch eine oder mehrere nicht benachbarte CH₂-Gruppen durch -O- und/oder -S- ersetzt sein können,
(b) 1,4-Phenylenrest, worin auch eine oder zwei CH-Gruppen durch N ersetzt sein können,
(c) Rest aus der Gruppe 1,4-Cyclohexenylen, 1,4-Bicyclo(2,2,2)-octylen, Piperidin-1,4-diyl, Naphthalin-2,6-diyl, Decahydronaphthalin-2,6-diyl und 1,2,3,4-Tetrahydronaphthalin-2,6-diyl,
wobei die Reste (a) und (b) durch CN oder Fluor substituiert sein können,
Z¹ jeweils unabhängig voneinander -CO-O-, -O-CO-, -CH₂O-, -OCH₂-, -CH₂CH₂-, -CH=CH-, -C≡C- oder eine Einfachbindung,
m 1, 2 oder 3,
Q Alkylen mit 2 bis 6 C-Atomen, worin auch eine CH₂-Gruppe durch -O-, -S-, -CO-O- oder -O-CO- ersetzt sein kann, -O-, -S-, -CH₂-, -CO-O-, -O-CO- oder eine Einfachbindung
bedeutet, mit der Maßgabe, daß Q Alkylen mit 2 bis 6 C-Atomen, worin auch eine CH₂-Gruppe durch -O-, -S-, -CO-O- oder -O-CO- ersetzt sein kann, -S-, -CH₂-, -CO-O-, -O-CO- oder eine Einfachbindung bedeutet, falls R-(A¹-Z¹)ₘ-A²- 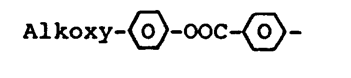 oder 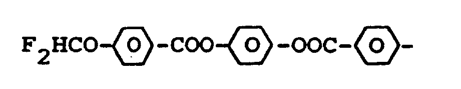 bedeutet.

2. Difluormethylverbindungen der Formel I nach Anspruch 1, dadurch gekennzeichnet, daß A² 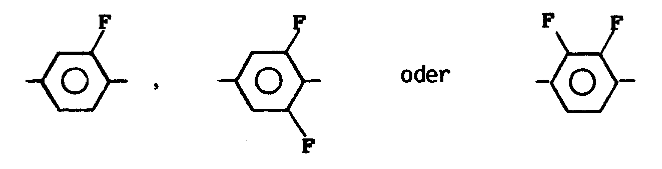 ist.

3. Difluormethylverbindungen der Formel I nach Anspruch 1 oder 2, dadurch gekennzeichnet, daß R ein geradkettiger Alkylrest mit 2 bis 7 C-Atomen ist.

4. Difluormethylverbindungen der Formel I nach mindestens einem der Ansprüche 1, 2 oder 3, dadurch gekennzeichnet, daß Q -O- bedeutet.

5. Verwendung von Verbindungen der Formel I als Komponenten flüssigkristalliner Medien.

6. Flüssigkristallines Medium mit mindestens zwei flüssigkristallinen Komponenten, dadurch gekennzeichnet, daß sie mindestens eine Verbindung der Formel I enthält.

7. Flüssigkristall-Anzeigeelement, dadurch gekennzeichnet, daß es ein flüssigkristallines Medium nach Anspruch 6 enthält.

8. Elektrooptisches Anzeigeelement, dadurch gekennzeichnet, daß es als Dielektrikum ein flüssigkristallines Medium nach Anspruch 6 enthält.

## Claims

1. Difluoromethyl compounds of the formula I
R-(A¹-Z¹)ₘ-A²-Q-CHF₂ I
in which
R is halogen, -CN, -NCS, a straight-chain alkyl or alkenyl radical having 1 to 15 carbon atoms, in which one or more CH₂ groups in these radicals, independently of one another, can each also be replaced by -O-, -S-, 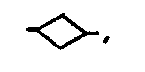 -CO-, -CO-O-, -O-CO- or -O-CO-O- in such a manner that oxygen atoms are not directly linked to one another, or a straight-chain perfluoroalkyl radical, in which one or more fluorine atoms can also be replaced by H,
A¹ and A², independently of one another, are each a
(a) trans-1,4-cyclohexylene radical in which one or more non-adjacent CH₂ groups can also be replaced by -O- and/or -S-,
(b) 1,4-phenylene radical in which one or two CH groups can also be replaced by N,
(c) radical from the group consisting of 1,4-cyclohexenylene, 1,4-bicyclo[2,2,2]-octylene, piperidine-1,4-diyl, naphthalene-2,6-diyl, decahydronaphthalene2,6-diyl and 1,2,3,4-tetrahydronaphthalene-2,6-diyl,
in which the radicals (a) and (b) can be substituted by CN or fluorine,
Z¹ independently of one another are each -CO-O, -O-CO-, -CH₂O-, -OCH₂-, -CH₂CH₂-, -CH=CH-, -C≡C- or a single bond,
m is 1, 2 or 3 and
Q is alkylene having 2 to 6 carbon atoms in which a CH₂ group can also be replaced by -O-, -S-, -CO-O- or -O-CO-, -O-, -S-, -CH₂-, -CO-O-, -O-CO- or a single bond,
with the proviso that Q is alkylene having 2 to 6 carbon atoms in which one CH₂ group can also be replaced by -O-, -S-, -CO-O- or -O-CO-, -S-, -CH₂-, -CO-O-, -O-CO- or a single bond, provided R-(A¹-Z¹)ₘ-A²- is alkoxy- 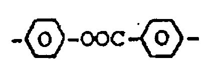 or

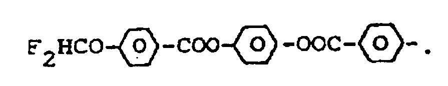

2. Difluoromethyl compounds of the formula I according to Claim 1, characterized in that A² is

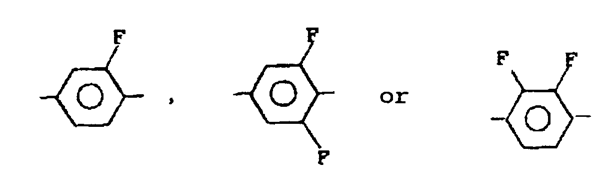

3. Difluoromethyl compounds of the formula I according to Claim 1 or 2, characterized in that R is a straight-chain alkyl radical having 2 to 7 carbon atoms.

4. Difluoromethyl compounds of the formula I according to at least one of Claims 1, 2 or 3, characterized in that Q is -O-.

5. Use of the compounds of the formula I as components of liquid-crystalline media.

6. Liquid-crystalline medium containing at least two liquid-crystalline components, characterized in that it contains at least one compound of the formula I.

7. Liquid crystal display element, characterized in that it contains a liquid-crystalline medium according to Claim 6.

8. Electrooptical display element, characterized in that it contains a liquid-crystalline medium according to Claim 6 as dielectric.

## Revendications

1. Composés difluorométhylés de formule I
R-(A¹-Z¹)ₘ-A²-Q-CHF₂ I
dans laquelle
R représente un halogène, un groupe -CN, -NCS, un groupe alkyle ou alcényle à chaîne droite ou ramifiée en C1-C15 dans lequel un ou plusieurs groupes CH₂ peuvent être remplacés, indépendamment les uns des autres, par -O-, -S-, 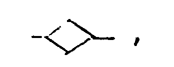 -CO-, -CO-O-, -O-CO- ou -O-CO-O- en sorte qu'il n'y ait pas de liaison directe entre des atomes d'oxygène, ou un groupe perfluoralkyle à chaîne droite dans lequel le fluor peut être remplacé partiellement par H,
A¹ et A² représentent chacun, indépendamment l'un de l'autre,
(a) un groupe trans-1,4-cyclohexylène dans lequel un ou plusieurs groupes CH₂ non voisins peuvent être remplacés par -O- et/ou -S-,
(b) un groupe 1,4-phénylène dans lequel un ou deux groupes CH peuvent être remplacés par N,
(c) un groupe choisi parmi les groupes 1,4-cyclohexénylène, 1,4-bicyclo(2,2,2)-octylène, pipéridine-1,4-diyle, naphtalène-2,6-diyle, décahydronaphtalène-2,6-diyle et 1,2,3,4-tétrahydronaphtalène-2,6-diyle,
les groupes (a) et (b) peuvent être substitués par CN ou le fluor,
chacun des symboles Z¹ représente, indépendamment, -CO-O-, -O-CO-, -CH₂O-, -OCH₂-, -CH₂CH₂-, -CH=CH-, -C≡C- ou une liaison simple,
m est égal à 1, 2 ou 3,
Q représente un groupe alkylène en C2-C6 dans lequel un groupe CH₂ peut être remplacé par -O-, -S-, -CO-O- ou -O-CO-, un pont -O-, -S-, -CH₂-, -CO-O-, -O-CO- ou une liaison simple,
sous réserve que Q représente un groupe alkylène en C2-C6 dans lequel un groupe CH₂ peut être remplacé par -O-, -S-, -CO-O- ou -O-CO-, un pont -S-, -CH₂-,-CO-O-, -O-CO- ou une liaison simple lorsque R-(A¹-Z¹)ₘ-A²- représente un groupe

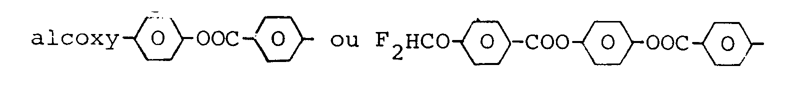

2. Composés difluorométhylés de formule I de la revendication 1, caractérisés en ce que A² représente

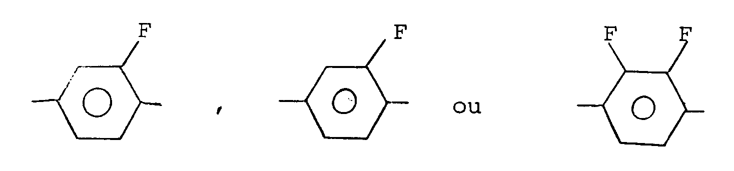

3. Composés difluorométhylés de formule I selon revendication 1 ou 2, caractérisés en ce que R représente un groupe alkyle à chaîne droite en C2-C7.

4. Composés difluorométhylés de formule I selon au moins une des revendications 1, 2 ou 3, caractérisés en ce que Q représente -O-.

5. Utilisation des composés de formule I en tant que composants de milieux à cristaux liquides.

6. Milieu à cristaux liquides à au moins deux composants à cristaux liquides, caractérisé en ce qu'il contient au moins un composé de formule I.

7. Elément d'affichage à cristaux liquides, caractérisé en ce qu'il contient un milieu à cristaux liquides selon revendication 6.

8. Elément d'affichage électro-optique caractérisé en ce qu'il contient en tant que diélectrique un milieu à cristaux liquides selon revendication 6.
